(19) **Europäisches Patentamt / European Patent Office / Office européen des brevets**

(11) **EP 4 755 872 A1**

(12) **EUROPEAN PATENT APPLICATION**
published in accordance with Art. 153(4) EPC

(43) Date of publication:
**10.06.2026 Bulletin 2026/24**

(21) Application number: **24849203.5**

(22) Date of filing: **31.07.2024**

(51) International Patent Classification (IPC):
**C07C 303/44** (2006.01)    **C07C 303/32** (2006.01)
**C07C 309/20** (2006.01)

(52) Cooperative Patent Classification (CPC):
**C07C 303/32; C07C 303/44; C07C 309/20**

(86) International application number:
**PCT/JP2024/027314**

(87) International publication number:
**WO 2025/028558 (06.02.2025 Gazette 2025/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.07.2023   JP 2023124883**

(71) Applicant: **Kao Corporation
Tokyo 103-8210 (JP)**

(72) Inventors:
• **YUASA,Kotaku
Tokyo 103-8210 (JP)**

• **FUJIOKA,Toku
Tokyo 103-8210 (JP)**
• **KAYAHARA,Jumpei
Tokyo 103-8210 (JP)**
• **NOMURA,Masato
Tokyo 103-8210 (JP)**
• **TSURUSAKO,Shuta
Tokyo 103-8210 (JP)**
• **YAMAUCHI,Hideaki
Tokyo 103-8210 (JP)**

(74) Representative: **Hoffmann Eitle
Patent- und Rechtsanwälte PartmbB
Arabellastraße 30
81925 München (DE)**

(54) **METHOD FOR PRODUCING DECOLORIZED INTERNAL OLEFIN SULFONATE COMPOSITION**

(57)    The present invention provides a method for producing a decolorized internal olefin sulfonate composition having a low odor, and good storage stability. A method for producing a decolorized internal olefin sulfonate composition of the present invention includes a decolorizing process (A) of performing an adjusting step (a1) of adjusting a concentration of an alkali compound in a system in which an internal olefin sulfonate composition containing an internal olefin sulfonate is present and a mixing step (a2) of mixing a decolorizing agent and the internal olefin sulfonate composition present in the system. The adjusting step (a1) is a step of adjusting the concentration of the alkali compound in the decolorizing process (A) so that the concentration of the alkali compound after mixing the decolorizing agent and the internal olefin sulfonate composition present in the system is 0.004 mmol or more and 0.26 mmol or less with respect to 1 g of the internal olefin sulfonate.

**Description**

TECHNICAL FIELD

The present invention relates to a method for producing a decolorized internal olefin sulfonate composition.

BACKGROUND ART

Conventionally, anionic surfactants, particularly alkyl sulfates and polyoxyalkylene alkyl ether sulfates, have been widely used as household and industrial washing components because of their excellent detergency and foaming power. As one of the anionic surfactants, a report has been made about an olefin sulfonate, particularly an internal olefin sulfonate obtained by using an internal olefin having a double bond not at any terminal of the olefin but at an inside thereof as a raw material.

The internal olefin sulfonate is generally obtained by sulfonating an internal olefin by reacting the internal olefin with sulfur trioxide, neutralizing the obtained sulfonated product, and further hydrolyzing the neutralized product.

The internal olefin sulfonate is usually obtained as an aqueous solution containing the internal olefin sulfonate (internal olefin sulfonate composition), but the internal olefin sulfonate composition is colored, and such coloring is disadvantageous when the internal olefin sulfonate composition is used as a detergent.

For the decolorization of the internal olefin sulfonate composition, for example, Patent Document 1 proposes a technique for decolorizing the internal olefin sulfonate composition with hydrogen peroxide.

PRIOR ART DOCUMENT

PATENT DOCUMENT

Patent Document 1: JP-A-2015-178467

SUMMARY OF THE INVENTION

PROBLEMS TO BE SOLVED BY THE INVENTION

However, it has been newly found that the internal olefin sulfonate composition decolorized with hydrogen peroxide may have stronger odor or lower storage stability.

The problems (odor and storage stability) of the internal olefin sulfonate composition decolorized with a decolorizing agent such as hydrogen peroxide have not been sufficiently studied heretofore, and the present invention provides a method for producing a decolorized internal olefin sulfonate composition having low odor and good storage stability.

MEANS FOR SOLVING THE PROBLEMS

The present invention relates to a method for producing a decolorized internal olefin sulfonate composition including a decolorizing process (A) of performing an adjusting step (a1) of adjusting a concentration of an alkali compound in a system in which an internal olefin sulfonate composition containing an internal olefin sulfonate is present and a mixing step (a2) of mixing a decolorizing agent and the internal olefin sulfonate composition present in the system, wherein the adjusting step (a1) is a step of adjusting the concentration of the alkali compound in the decolorizing process (A) so that the concentration of the alkali compound after mixing the decolorizing agent and the internal olefin sulfonate composition present in the system is 0.004 mmol or more and 0.26 mmol or less with respect to 1 g of the internal olefin sulfonate.

EFFECT OF THE INVENTION

According to a production method of the present invention, by adjusting the concentration of an alkali compound in a system to a specific range when a decolorizing agent and an internal olefin sulfonate composition are mixed, a decolorized internal olefin sulfonate composition having a good color fading effect, low odor, and good storage stability can be obtained.

MODE FOR CARRYING OUT THE INVENTION

Hereinafter, the present invention will be described in detail.

A method for producing a decolorized internal olefin sulfonate composition of the present invention includes:

a decolorizing process (A) of performing an adjusting step (a1) of adjusting a concentration of an alkali compound in a system in which an internal olefin sulfonate composition containing an internal olefin sulfonate is present and a mixing step (a2) of mixing a decolorizing agent and the internal olefin sulfonate composition present in the system, wherein the adjusting step (a1) is a step of adjusting the concentration of the alkali compound in the decolorizing process (A) so that the concentration of the alkali compound after mixing the decolorizing agent and the internal olefin sulfonate composition present in the system is 0.004 mmol or more and 0.26 mmol or less with respect to 1 g of the internal olefin sulfonate.

In the present invention, the internal olefin sulfonate is a sulfonate obtained by sulfonating, neutralizing, and hydrolyzing an internal olefin. That is, when the internal olefin is sulfonated, the internal olefin is changed to β-sultone, γ-sultone, and olefin sulfonic acid. Furthermore, these are converted into hydroxysulfonate and olefin sulfonate in a neutralization process and a hydrolysis process. The obtained product is mainly a mixture of these. In the present invention, these products, and mixtures thereof are collectively referred to as an internal olefin sulfonate.

The internal olefin sulfonate composition is an aqueous solution containing the internal olefin sulfonate, and is obtained through the following sulfonation process, neutralization process, and optional hydrolysis process.

<Sulfonation process>

The sulfonation process is a step of obtaining a sulfonated product by reacting the internal olefin with sulfur trioxide gas.

The internal olefin is an olefin having a double bond other than the terminal of an olefin chain, that is, an olefin having a double bond at a position of 2 or more of a carbon chain. The internal olefin may contain a trace amount of a so-called α-olefin in which the position of the double bond is at the 1-position of the carbon chain.

The number of carbon atoms in the internal olefin is preferably 10 or more, more preferably 12 or more, still more preferably 14 or more, and yet still more preferably 16 or more, and preferably 22 or less, and more preferably 18 or less, from the viewpoint of washing performance and the like when an internal olefin sulfonate is used as a detergent.

The internal olefins may be used alone or in combination of two or more. The internal olefin is at least one selected from the group consisting of an internal olefin having 16 carbon atoms and an internal olefin having 18 carbon atoms from the viewpoint of the washing performance and the like when the internal olefin sulfonate is used as a detergent. When two or more kinds of internal olefins are used in combination, the internal olefin having 16 carbon atoms and the internal olefin having 18 carbon atoms are preferably used in combination from the viewpoint of the washing performance and the like when the internal olefin sulfonate is used as the detergent.

The internal olefin can be produced by a known method, for example, a method described in WO 2011/052732 A.

The sulfur trioxide gas is not particularly limited, but is preferably diluted with air and an inert gas such as nitrogen. The concentration of the sulfur trioxide gas is preferably 0.5 vol% or more, and more preferably 1.0 vol% or more, from the viewpoint of efficiently reacting the internal olefin with the sulfur trioxide gas to reduce a reactor volume, thereby improving the economic efficiency. The concentration is preferably 10 vol% or less, and more preferably 6.0 vol% or less, from the viewpoint of appropriately controlling the reaction rate to obtain a sulfonated product having a good hue.

The amount of sulfur trioxide gas used is preferably 0.8 mol or more, more preferably 0.9 mol or more, and still more preferably 0.95 mol or more, based on 1 mol of the internal olefin, from the viewpoint of improving the yield of the sulfonated product, and is preferably 1.2 mol or less, more preferably 1.10 mol or less, and still more preferably 1.05 mol or less, from the viewpoint of the economic efficiency thereof and from the viewpoint of suppressing the coloring of the sulfonated product.

In the sulfonation process, a sulfonation reactor used is not particularly limited, but sulfonation reactor preferably includes an external jacket from the viewpoint of controlling a reaction temperature to obtain a sulfonated product having good quality. From the viewpoint of efficiently reacting a liquid internal olefin with the sulfur trioxide gas, a falling thin-film type sulfonation reactor is preferable in which the internal olefin flows down in a thin-film form and reacts with the sulfur trioxide gas. Examples of such a sulfonation reactor include reactors described in JP-A-11-315061, JP-B-49-48409, IT-A-1248079, and US-A-3257175.

A treatment temperature in the sulfonation process is preferably 0°C or higher from the viewpoint of preventing the coagulation of sulfur trioxide and the sulfonated product, and is preferably 50°C or lower from the viewpoint of suppressing the coloring of the sulfonated product.

Since the sulfonation reaction is an exothermic reaction, it is preferable to provide an external jacket in the sulfonation reactor and pass cooling water thereinto to cool the sulfonation reactor. The temperature of the cooling water, which is passed into the external jacket of the sulfonation reactor is preferably 0 °C or higher from the viewpoint of improving the reaction rate. The temperature is preferably 30°C or lower, and more preferably 20°C or lower, from the viewpoint of suppressing side reactions and thereby reducing the impurities such as the internal olefin and inorganic salts in the finally obtained internal olefin sulfonate.

An inert gas may be introduced between the internal olefin and the sulfur trioxide gas in order to control the reaction rate to suppress the deterioration of the quality of the sulfonated product. Examples of such a reactor include reactors described in JP-A-11-315061 and JP-B-49-48409.

The reaction rate of the internal olefin in the sulfonation reaction is preferably 95% or more, more preferably 96% or more, and still more preferably 97% or more, from the viewpoint of improving the yield of the sulfonated product. The reaction rate is preferably 99.8% or less from the viewpoint of suppressing the coloration of the sulfonated product due to excessive sulfur trioxide.

<Neutralization process>

The neutralization process is the step of neutralizing the sulfonated product obtained in the sulfonation process to obtain a neutralized product.

The alkali compound used for neutralization may be an inorganic alkali compound or an organic alkali compound. As the inorganic alkali compound, for example, alkali metal hydroxides such as sodium hydroxide and potassium hydroxide, and alkali metal carbonates such as sodium carbonate and potassium carbonate can be used. As the organic alkali compound, for example, ammonia, and amine compounds having 1 to 6 carbon atoms such as 2-aminoethanol can be used.

The alkali compound used for neutralization is preferably an inorganic alkali compound, more preferably an alkali metal hydroxide, and still more preferably at least one selected from sodium hydroxide and potassium hydroxide, from the viewpoint of the availability thereof and economic efficiency.

The alkali compound used for neutralization is preferably used as an aqueous alkali solution from the viewpoint of the ease of handling. In the neutralization, the sulfonated product and the aqueous alkali solution may be mixed, or the sulfonated product, the aqueous alkali solution, and water may be mixed. When the sulfonated product, the aqueous alkali solution, and water are mixed, an aqueous alkali solution having a high concentration can be used. The concentration of the aqueous alkali solution or the concentration of a mixed solution when the aqueous alkali solution and water are mixed is preferably 1% by mass or more, more preferably 4.5% by mass or more, still more preferably 7% by mass or more, yet still more preferably 10% by mass or more, and yet still more preferably 12% by mass or more, from the viewpoint of improving the economic efficiency and the viewpoint of suppressing the production of impurities such as the internal olefin and inorganic salts. The concentration is preferably 50% by mass or less, more preferably 32% by mass or less, still more preferably 23% by mass or less, yet still more preferably 20% by mass or less, and yet still more preferably 18% by mass or less, from the viewpoint of improving the productivity in the hydrolysis process.

In the present invention, it is preferable to control the concentration of the alkali compound in the internal olefin sulfonate composition after the hydrolysis process by adjusting the amount of the alkali compound used in the neutralization process. The amount of the alkali compound used in the neutralization process is preferably 1 time by mole or more, more preferably 1.02 times by mole or more, and still more preferably 1.030 times by mole or more, with respect to a sulfonic acid group, from the viewpoint of suppressing the production of impurities such as the internal olefin and inorganic salts and the viewpoint of improving the reactivity. The amount is more preferably 1.034 times by mole or more, more preferably 1.035 times by mole or more, and still more preferably 1.04 times by mole or more, from the viewpoint of suppressing the odor deterioration after decolorizing. The amount is preferably 1.13 times by mole or less, more preferably 1.12 times by mole or less, still more preferably 1.110 times by mole, yet still more preferably 1.105 times by mole or less, yet still more preferably 1.10 times by mole or less, yet still more preferably 1.09 times by mole or less, yet still more preferably 1.08 times by mole or less, yet still more preferably 1.07 times by mole or less, and yet still more preferably 1.06 times by mole or less, from the viewpoint of improving the economic efficiency and the viewpoint of improving the storage stability.

In the neutralization process, a temperature when the sulfonate is mixed with the aqueous alkali solution and a temperature during the neutralization reaction are preferably 40°C or lower, more preferably 35°C or lower, still more preferably 30°C or lower, and yet still more preferably 25°C or lower, from the viewpoint of suppressing the production of impurities such as the internal olefin and inorganic salts, due to side reactions, and are preferably 0°C or higher, more preferably 10°C or higher, still more preferably 15°C or higher, and yet still more preferably 20°C or higher, from the viewpoint of improving the reactivity.

In the neutralization process, any type of mixer may be used as long as the mixer can efficiently mix the sulfonated product with the aqueous alkali solution and can apply a shear force to the oily product. Examples of the mixer include a stationary type mixer, a collision type mixer, an agitating impeller type mixer, and a vibration type mixer. Examples of the stationary type mixer include a static mixer manufactured by Noritake Co., Ltd. Examples of the collision type mixer include a high-pressure emulsifying machine manufactured by Nanomizer Inc. Examples of the agitating impeller type mixer include a Milder manufactured by Pacific Machinery & Engineering Co., Ltd., and a Homo Mixer manufactured by PRIMIX Corporation. Among them, the agitating impeller type mixer is preferable from the viewpoint of equipment costs.

When an agitating impeller type mixer is used, the agitating speed of the agitator is preferably 5 m/s or more, more preferably 10 m/s or more, and still more preferably 20 m/s or more, from the viewpoint of suppressing the production of impurities such as the internal olefin and inorganic salts. The agitating speed is preferably 200 m/s or less, more preferably 100 m/s or less, still more preferably 50 m/s or less, yet still more preferably 30 m/s or less, yet still more preferably 27.5 m/s or less, and yet still more preferably 25 m/s or less, from the viewpoint of suppressing the generation of heat. Here, the agitating speed is the speed of the tip of the agitating impeller, and is represented by $2\times$(pi)$\times$(radius of agitating impeller)$\times$(number of rotations per unit time).

The neutralization process is performed in a continuous method or a batch method or the like, and from the viewpoint of suppressing the production of by-products and improving the productivity, it is preferable to perform the neutralization process in a so-called continuous method in which a sulfonated product and an aqueous alkali solution are added while a reaction liquid is circulated using a loop type reaction device, and the reaction liquid is simultaneously extracted. Here, the "batch method" is a process in which a reaction is started and progressed after a raw material is charged into a reactor, and a product is recovered after the completion, and the "continuous method" is a process in which both raw material charging and product recovery are simultaneously performed during the progress of the reaction. The "semi-batch method" described later is a process in which either raw material charging or product recovery is simultaneously performed during the progress of the reaction.

In the neutralization process, a mixing time when the sulfonated product and the aqueous alkali solution are mixed is preferably 10 minutes or more, more preferably 20 minutes or more, and still more preferably 30 minutes or more, from the viewpoint of reducing the impurities. The mixing time is preferably 120 minutes or less, more preferably 90 minutes or less, still more preferably 60 minutes or less, and yet still more preferably 50 minutes or less, from the viewpoint of improving the economic efficiency.

The concentration of the internal olefin sulfonate in the neutralization product is preferably 40% by mass or more, more preferably 45% by mass or more, and still more preferably 48% by mass or more, from the viewpoint of obtaining a high concentration internal olefin sulfonate composition. The concentration is preferably 65% by mass or less, and more preferably 60% by mass or less, from the viewpoint of suppressing the increase of the viscosity of the neutralization product. The concentration of the internal olefin sulfonate in the neutralization product is a value calculated by concentration of internal olefin sulfonate + concentration of $\gamma$-sultone$\times$(molecular weight of 3-hydroxysulfonate/molecular weight of $\gamma$-sultone).

<Hydrolysis process>

The hydrolysis process is a step of hydrolyzing the neutralization product obtained in the neutralization process by heating to obtain a hydrolyzate.

In the hydrolysis process, a temperature during the hydrolysis is preferably 120°C or higher, more preferably 140°C or higher, and still more preferably 160°C or higher, from the viewpoint of improving the reactivity, and is preferably 220°C or lower, and more preferably 200°C or lower, from the viewpoint of suppressing the decomposition of the product.

The hydrolysis reaction may be conducted in a batch reactor or in a continuous reactor. When the batch reactor is used, it is preferable to mix using an agitator or the like.

The treatment time of the hydrolysis process is preferably 30 minutes or more, more preferably 45 minutes or more, and still more preferably 1 hour or more, from the viewpoint of completing the reaction, and is preferably 4 hours or less, and more preferably 3 hours or less, from the viewpoint of improving the productivity.

The average double bond position of the raw material olefin of the internal olefin sulfonate is preferably 3 or more, more preferably 3.5 or more, and still more preferably 3.8 or more, from the viewpoint of the color of the sulfonate composition obtained by reacting and the color of the sulfonate composition obtained after decolorizing. The average double bond position is preferably 5 or less, more preferably 4.5 or less, still more preferably 4.4 or less, and yet still more preferably 4.3 or less, from the viewpoint of suppressing the contamination of the amount of by-products and reducing the peroxide value of the sulfonate composition obtained after decolorizing.

The internal olefin sulfonate preferably contains one or more internal olefin sulfonates selected from the group consisting of an internal olefin sulfonate having 16 carbon atoms and an internal olefin sulfonate having 18 carbon atoms, from the viewpoint of the washing performance and the like when the decolorized internal olefin sulfonate composition (decolorized internal olefin sulfonate composition) obtained through a decolorizing process (A) described later is used as a detergent. Here, the number of carbon atoms of the internal olefin sulfonate is derived from the carbon chain of the internal olefin.

From the viewpoint of suppressing the deterioration of the odor of the internal olefin sulfonate composition after the decolorizing process (A) and the viewpoint of reducing the peroxide value of the internal olefin sulfonate composition after the decolorizing process (A), the concentration of the alkali compound in the internal olefin sulfonate composition obtained in the hydrolysis process is preferably 0.010 mmol or more, more preferably 0.015 mmol or more, still more preferably 0.020 mmol or more, yet still more preferably 0.025 mmol or more, and yet still more preferably 0.029 mmol or more, with respect to 1 g of the internal olefin sulfonate in the internal olefin sulfonate composition. From the viewpoint of suppressing the decrease of the storage stability, the concentration is preferably 0.27 mmol or less, more preferably 0.240 mmol or less, and still more preferably 0.210 mmol or less. From the viewpoint of increasing the fading rate, the concentration is preferably 0.27 mmol or less, more preferably 0.240 mmol or less, still more preferably 0.220 mmol or less, still more preferably 0.210 mmol or less, still more preferably 0.20 mmol or less, still more preferably 0.18 mmol or less, still more preferably 0.16 mmol or less, still more preferably 0.10 mmol or less, and still more preferably 0.08 mmol or less.

The hydrolysis process is not limited to the step of obtaining the hydrolyzate by hydrolysis, and the concentration of the alkali compound in the internal olefin sulfonate composition may be controlled by adjusting the amount of the alkali compound.

From the viewpoint of reducing the amount of impurities in the internal olefin sulfonate composition, the internal olefin sulfonate composition is preferably produced by a production method in which the concentration of the alkali compound in the internal olefin sulfonate composition after the hydrolysis process is controlled within the suitable range by an adjusting method selected from a method of adjusting the amount of the alkali compound used in the neutralization process and a method of adjusting the amount of the alkali compound in the hydrolysis process. From the same viewpoint, it is more preferable that the internal olefin sulfonate composition is produced by a production method in which the concentration of the alkali compound in the internal olefin sulfonate composition after the hydrolysis process is controlled within the suitable range by a method of adjusting the amount of the alkali compound used in the neutralization process. The suitable range of the amount of the alkali compound used in the neutralization process is as described above.

<Decolorizing process (A)>

The decolorizing process (A) is a step of performing an adjusting step (a1) of adjusting a concentration of an alkali compound in a system in which an internal olefin sulfonate composition containing an internal olefin sulfonate is present and a mixing step (a2) of mixing a decolorizing agent and the internal olefin sulfonate composition present in the system. The adjusting step (a1) is a step of adjusting the concentration of the alkali compound in the decolorizing process (A) so that the concentration of the alkali compound after mixing the decolorizing agent and the internal olefin sulfonate composition present in the system is 0.004 mmol or more and 0.26 mmol or less with respect to 1 g of the internal olefin sulfonate.

Here, in the present invention, the "reactor" refers to a reaction tank itself used in a batch method or a semi-batch method, the "reaction device" refers to the entire unit involved in the reaction including the reaction tank, piping, and the like, and the reaction device in a continuous method may not include the reaction tank.

The system in which the internal olefin sulfonate composition is present may be a liquid (liquid composition) in which the internal olefin sulfonate composition is present.

The order of operating the adjusting step (a1) and the mixing step (a2) is not particularly limited, and the adjusting step (a1) may be performed after the mixing step (a2), or the mixing step (a2) may be performed after the adjusting step (a1). The mixing step (a2) and the adjusting step (a1) may be performed in parallel. However, a decolorizing treatment (decolorizing reaction) is started by mixing (contacting) the decolorizing agent and the internal olefin sulfonate composition, and thus when the adjusting step (a1) is performed after the mixing step (a2), it is preferable to perform the adjusting step (a1) immediately after the mixing step (a2) from the viewpoint of suppressing the deterioration of odor. In the present invention, it is preferable to perform the mixing step (a2) and the adjusting step (a1) in parallel from the viewpoint of suppressing the deterioration of odor and the viewpoint of improving the economic efficiency.

The decolorizing treatment (decolorizing reaction) refers to a state where the decolorizing agent and the internal olefin sulfonate composition coexist, and the decolorizing treatment includes the mixing step (a2) and an aging step (b) described later. Here, the aging step (b) is a step of maintaining the state where the decolorizing agent and the internal olefin sulfonate composition coexist after mixing the decolorizing agent.

The decolorizing process (A) may be performed at any stage after the internal olefin sulfonate composition is formed in the step of producing the internal olefin sulfonate composition, but is preferably performed after the neutralization process or after the hydrolysis process, and is more preferably performed after the hydrolysis process from the viewpoint of suppressing the generation of by-products.

The suitable aspect of the concentration of the alkali compound in the internal olefin sulfonate composition before the decolorizing process (A) is performed is the same as the suitable aspect of the concentration of the alkali compound in the internal olefin sulfonate composition obtained in the hydrolysis process.

The system in which the internal olefin sulfonate composition containing the internal olefin sulfonate is present is a system containing the internal olefin sulfonate composition before decolorizing treatment, and specific examples thereof include a system containing the internal olefin sulfonate composition after the neutralization process and a system containing the internal olefin sulfonate composition after the hydrolysis process. Each step will be described below.

[Adjusting step (a1)]

In the adjusting step (a1), the concentration of the alkali compound is adjusted in the decolorizing process (A) so that the concentration of the alkali compound after mixing the decolorizing agent and the internal olefin sulfonate composition present in the system is 0.004 mmol or more and 0.26 mmol or less with respect to 1 g of the internal olefin sulfonate. By mixing the decolorizing agent and the internal olefin sulfonate composition to perform the decolorizing reaction, the concentration of the alkali compound in the reaction system is reduced, and thus the concentration of the alkali compound in the system is controlled (adjusted) in the adjusting step (a1). In the present invention, it is preferable to adjust the concentration of the alkali compound in the system immediately after the mixing step (a2) to be in the above range.

In the adjusting step (a1), the concentration of the alkali compound in the system is adjusted to 0.004 mmol or more and 0.26 mmol or less with respect to 1 g of the internal olefin sulfonate after the mixing step (a2). By setting the concentration of such an alkali compound, the decolorized internal olefin sulfonate composition having a good color fading effect, low odor, and good storage stability is obtained. The concentration of the alkali compound is preferably 0.005 mmol or more from the viewpoint of suppressing the deterioration of odor and the viewpoint of reducing the peroxide value. The concentration is more preferably 0.010 mmol or more, still more preferably 0.015 mmol or more, still more preferably 0.020 mmol or more, still more preferably 0.030 mmol or more, from the viewpoint of reducing the peroxide value. The concentration of the alkali compound is preferably 0.23 mmol or less, more preferably 0.215 mmol or less, and still more preferably 0.20 mmol or less, from the viewpoint of suppressing the decrease of the storage stability of the decolorized internal olefin sulfonate composition. The concentration is preferably 0.23 mmol or less, more preferably 0.215 mmol or less, still more preferably 0.20 mmol or less, yet still more preferably 0.15 mmol or less, yet still more preferably 0.10 mmol or less, yet still more preferably 0.08 mmol or less, and yet still more preferably 0.06 mmol or less, from the viewpoint of increasing the fading rate. Since the concentration of the alkali compound decreases during the decolorizing treatment, it is preferable to adjust the concentration of the alkali compound immediately after the decolorizing treatment (that is, immediately after the mixing step (a2)). The adjusting step (a1) is a step of adjusting the concentration of the alkali compound, and when the concentration of the alkali compound is already within the suitable range, the operation of changing the concentration of the alkali compound may not be performed. The case where it is not necessary to adjust the concentration of the alkali compound is also included in the "adjusting step (a1)" of the present invention.

When the adjusting step (a1) is performed after the mixing step (a2), the concentration of the alkali compound may be adjusted in the adjusting step (a1). Meanwhile, when the adjusting step (a1) is provided before the mixing step (a2), the concentration of the alkali compound is preferably adjusted in consideration of the concentration of the alkali compound decreased by the decolorizing treatment. Specifically, the concentration of the alkali compound in the adjusting step (a1) is preferably 0.010 mmol or more, more preferably 0.015 mmol or more, more preferably 0.020 mmol or more, still more preferably 0.025 mmol or more, still more preferably 0.030 mmol or more, and still more preferably 0.035 mmol or more, with respect to 1 g of the internal olefin sulfonate, from the viewpoint of suppressing the deterioration of odor and the viewpoint of reducing the peroxide value. From the viewpoint of suppressing the decrease of the storage stability, the concentration is preferably 0.27 mmol or less, more preferably 0.240 mmol or less, and more preferably 0.210 mmol or less. From the viewpoint of increasing the fading rate, the concentration is still more preferably 0.240 mmol or less, yet still more preferably 0.220 mmol or less, yet still more preferably 0.210 mmol or less, yet still more preferably 0.20 mmol or less, yet still more preferably 0.18 mmol or less, yet still more preferably 0.16 mmol or less, yet still more preferably 0.10 mmol or less, and more preferably 0.08 mmol or less.

The adjusting method is not particularly limited, but the adjusting method can be performed by extracting the liquid in the system and analyzing the concentration of the alkali compound in the extracted liquid promptly at room temperature. As a result of this analysis, when the concentration of the alkali compound after the mixing step is lower than the suitable concentration range, such as when the concentration is less than 0.004 mmol with respect to 1 g of the internal olefin sulfonate, the concentration of the alkali compound after the mixing step can be adjusted to be the suitable concentration of the alkali compound by adding the alkali compound into the system.

The alkali compound may be an inorganic alkali compound or an organic alkali compound. Examples of the inorganic alkali compound include alkali metal hydroxides such as sodium hydroxide and potassium hydroxide, and alkali metal carbonates such as sodium carbonate and potassium carbonate. Examples of the organic alkali compound include ammonia, and amine compounds having 1 or more and 6 or less carbon atoms such as 2-aminoethanol.

The alkali compound is preferably an inorganic alkali compound, more preferably an alkali metal hydroxide, and still more preferably at least one selected from the group consisting of sodium hydroxide and potassium hydroxide, from the viewpoint of the easy availability and the improvement of the economic efficiency.

The alkali compound is preferably used as an aqueous alkali solution from the viewpoint of the ease of handling.

The adjusting step (a1) can be performed in a batch, semi-batch, or continuous method, and is preferably performed in a continuous method from the viewpoint of improving the productivity.

In the adjusting step (a1), for example, when the loop type reaction device is used, the liquid in the system is extracted, and the concentration of the alkali compound in the extracted mixed solution can be quickly analyzed at room temperature, which is preferable.

In the adjusting step (a1), from the viewpoint of the odor reduction, the adjustment is preferably performed in a continuous method with a circulation system, and for example, the adjustment is preferably performed in a continuous method while the internal olefin sulfonate composition is circulated using the loop type reaction device or the like. A circulation ratio is preferably greater than 0 times, more preferably 1 time or more, more preferably 2 times or more, still more preferably 3 times or more, and still more preferably 4 times or more, from the viewpoint of the odor reduction. The circulation ratio is preferably 20 times or less, more preferably 10 times or less, and still more preferably 8 times or less, from the viewpoint of the improvement of the economic efficiency. Here, the circulation ratio is a ratio of the flow rate circulating in the reaction device to the flow rate charged into the reaction device, and is represented by ((the flow rate in the reaction device)/(the flow rate charged into the reaction device))-1. Here, the flow rate in the reaction device indicates the flow rate immediately after joining with the fluid charged into the reaction device.

In the adjusting step (a1), any type of mixer may be used as long as the mixer can efficiently mix the internal olefin sulfonate composition and the decolorizing agent (aqueous solution of decolorizing agent). Examples of the mixer include an agitating impeller, a stationary type mixer, a collision type mixer, an agitating impeller type mixer, and a vibration type mixer. Examples of the agitating impeller include a small impeller and a large impeller, and examples of the small impeller include a flat paddle impeller, an inclined paddle impeller, and a propeller impeller. Examples of the large impeller include an anchor impeller, a helical ribbon impeller, a screw impeller, Maxblend, a super mix impeller (MR205 (product name, manufactured by Satake MultiMix Corporation) and the like), and Fullzone. Examples of the stationary type mixer include a static mixer manufactured by Noritake Co., Ltd. Examples of the collision type mixer include a high-pressure emulsifying machine manufactured by Nanomizer Inc. Examples of the agitating impeller type mixer include MILDER manufactured by Pacific Machinery & Engineering Co., Ltd., CAVITRON manufactured by Pacific Machinery & Engineering Co., Ltd., and PIPELINE-HOMO MIXER manufactured by PRIMIX Corporation. Among them, from the viewpoint of improving the color fading effect, an agitating impeller or an agitating impeller type mixer is preferable, a large impeller or an agitating impeller type mixer is more preferable, and an agitating impeller type mixer is still more preferable.

When a reaction tank having the agitating impeller or an agitating impeller type mixer is used in the adjusting step (a1), the peripheral speed of the agitating impeller is preferably 0.01 m/s or more, more preferably 0.05 m/s or more, still more preferably 0.1 m/s or more, yet still more preferably 0.3 m/s or more, yet still more preferably 0.9 m/s or more, yet still more preferably 4.7 m/s or more, yet still more preferably 7.0 m/s or more, and yet still more preferably 9.0 m/s or more, from the viewpoint of improving the decolorizing effect (fading rate) and the mixing properties. The peripheral speed is preferably 50 m/s or less, more preferably 40 m/s or less, still more preferably 26 m/s or less, and yet still more preferably 15 m/s or less, from the viewpoint of improving the economic efficiency. When the adjusting step (a1) is performed in a continuous method, the peripheral speed of the agitating impeller is preferably 0.1 m/s or more, more preferably 0.3 m/s or more, still more preferably 4 m/s or more, yet still more preferably 7.0 m/s or more, and yet still more preferably 9.0 m/s or more, from the viewpoint of increasing the fading rate. The peripheral speed is preferably 50 m/s or less, more preferably 40 m/s or less, more preferably 26 m/s or less, and still more preferably 15 m/s or less, from the viewpoint of reducing the odor deterioration and improving the economic efficiency. When the adjusting step (a1) is performed in a semi-batch method, the peripheral speed of the agitating impeller is preferably 0.1 m/s or more, more preferably 0.3 m/s or more, still more preferably 0.5 m/s or more, and yet still more preferably 0.7 m/s or more, from the viewpoint of increasing the fading rate. The peripheral speed is preferably 50 m/s or less, more preferably 40 m/s or less, still more preferably 26 m/s or less, yet still more preferably 15 m/s or less, yet still more preferably 10 m/s or less, and yet still more preferably 8 m/s or less, from the viewpoint of reducing the odor deterioration and improving the economic efficiency. When the adjusting step (a1) is performed in a batch method, the peripheral speed of the agitating impeller is preferably 0.1 m/s or more, more preferably 0.3 m/s or more, still more preferably 0.9 m/s or more, yet still more preferably 1.0 m/s or more, and yet still more preferably 1.5 m/s or more, from the viewpoint of increasing the fading rate. The peripheral speed is preferably 50 m/s or less, more preferably 40 m/s or less, still more preferably 26 m/s or less, still more preferably 15 m/s or less, still more preferably 10 m/s or less, still more preferably 5 m/s or less, and still more preferably 3 m/s or less, from the viewpoint of reducing the odor deterioration and improving the economic efficiency.

When the reaction tank having an agitating impeller, or the agitating impeller type mixer is used in the adjusting step (a1), a ratio (d/D) of an impeller diameter d to a tank inner diameter D is preferably 0.01 or more, more preferably 0.05 or more, still more preferably 0.1 or more, yet still more preferably 0.3 or more, yet still more preferably 0.4 or more, yet still more preferably 0.5 or more, and yet still more preferably 0.55 or more, from the viewpoint of improving the production capacity, improving the mixing properties, and increasing the fading rate. The ratio (d/D) is preferably less than 1, more preferably 0.9 or less, still more preferably 0.8 or less, and yet still more preferably 0.7 or less, from the viewpoint of improving the economic efficiency. The ratio (d/D) is preferably 0.7 or more, more preferably 0.8 or more, still more preferably 0.9 or more, and preferably less than 1, from the viewpoint of improving the mixing properties.

When the reaction tank having an agitating impeller, or the agitating impeller type mixer is used in the adjusting step (a1), a ratio (h/H) of an agitating impeller height h to a tank height H is preferably 0.01 or more, more preferably 0.05 or more, still more preferably 0.1 or more, yet still more preferably 0.3 or more, and yet still more preferably 0.4 or more, from the viewpoint of improving the mixing properties and increasing the fading rate. The ratio (h/H) is preferably less than 1, more preferably 0.9 or less, still more preferably 0.8 or less, and yet still more preferably 0.7 or less, from the viewpoint of suppressing the foaming. The ratio (h/H) is preferably 0.7 or more, more preferably 0.8 or more, still more preferably 0.9 or more, and preferably less than 1, from the viewpoint of improving the mixing properties.

When the reaction tank having agitating impellers or the agitating impeller type mixer is used in the adjusting step (a1), the number of stages of the agitating impellers is preferably 1 or more, more preferably 2 or more, and still more preferably 3 or more, from the viewpoint of improving the mixing properties. The number of stages is preferably 10 or less, more preferably 9 or less, and still more preferably 8 or less, from the viewpoint of improving the economic efficiency.

When the adjustment is performed in a batch method using the reaction tank having agitating impellers in the adjusting step (a1), the d/D, h/H and the number of stages of the agitating impellers are preferably in the above suitable ranges.

The adjusting step (a1) is preferably performed under shear conditions. The shear rate is preferably 5/s or more, more preferably 10/s or more, still more preferably 100/s or more, yet still more preferably 500/s or more, yet still more preferably 1,000/s or more, and yet still more preferably 1,500/s or more, from the viewpoint of improving the mixing properties. The shear rate is preferably 100,000/s or less, more preferably 50,000/s or less, still more preferably 30,000/s or less, and yet still more preferably 20,000/s or less, from the viewpoint of reducing the odor deterioration and improving the economic efficiency. When the adjusting step (a1) is performed in a continuous method, the shear rate is preferably 100/s or more, more preferably 500/s or more, still more preferably 1,000/s or more, and still more preferably 1,500/s or more, from the viewpoint of increasing the fading rate. The shear rate is preferably 100,000/s or less, more preferably 50,000/s or less, still more preferably 30,000/s or less, and yet still more preferably 20,000/s or less, from the viewpoint of reducing the odor deterioration and improving the economic efficiency. When the adjusting step (a1) is performed in a semi-batch method, the shear rate is preferably 10/s or more, more preferably 100/s or more, still more preferably 500/s or more, yet still more preferably 1,000/s or more, and yet still more preferably 1,500/s or more, from the viewpoint of increasing the fading rate. The shear rate is preferably 100,000/s or less, more preferably 50,000/s or less, still more preferably 40,000/s or less, yet still more preferably 30,000/s or less, yet still more preferably 20,000/s or less, yet still more preferably 10,000/s or less, and yet still more preferably 8,000/s or less, from the viewpoint of reducing the odor deterioration and improving the economic efficiency. When the adjusting step (a1) is performed in a batch method, the shear rate is preferably 5/s or more, more preferably 8/s or more, still more preferably 10/s or more, and yet still more preferably 12/s or more, from the viewpoint of increasing the fading rate. The shear rate is preferably 5,000/s or less, more preferably 1,000/s or less, still more preferably 500/s or less, yet still more preferably 100/s or less, and yet still more preferably 50/s or less, from the viewpoint of reducing the odor deterioration and improving the economic efficiency. Here, shear rate (1/s)=number of rotations of (rpm) of agitating impeller×pi×impeller diameter (m)/(30×(reaction tank inner diameter (m)- impeller diameter (m))) is set.

Stirring power per unit liquid amount (Pv) of the mixer used in the adjusting step (a1) is preferably 0.1 kW/m$^3$ or more, more preferably 0.2 kW/m$^3$ or more, and still more preferably 0.3 kW/m$^3$ or more, from the viewpoint of improving the mixing properties. The power is preferably 100 kW/m$^3$ or less, more preferably 50 kW/m$^3$ or less, still more preferably 30 kW/m$^3$ or less, yet still more preferably 10 kW/m$^3$ or less, yet still more preferably 5 kW/m$^3$ or less, and yet still more preferably 1 kW/m$^3$ or less, from the viewpoint of improving the economic efficiency. Stirring power per unit liquid amount (Pv) (kW/m$^3$) =stirring power P (kW)/liquid amount (m$^3$) is set. Here, the stirring power P represents a value obtained by subtracting idling power from power in an actual liquid.

The pressure in the system in the adjusting step (a1) is preferably 0 MPaG or more, more preferably 0.01 MPaG or more, more preferably 0.05 MPaG or more, still more preferably 0.1 MPaG or more, and still more preferably 0.2 MPaG or more, from the viewpoint of the pH controllability, the improvement of the mixing properties, and the suppression of the foaming. The pressure is preferably 10 MPaG or less, more preferably 5 MPaG or less, still more preferably 1 MPaG or less, and still more preferably 0.5 MPaG or less, from the viewpoint of improving the economic efficiency. Here, G of MPaG indicates a gauge pressure.

[Mixing step (a2)]

The decolorizing agent is not particularly limited, but an oxidizing agent is preferable. Examples of the oxidizing agent include hydrogen peroxide, hypochlorous acid, nitric acid, potassium nitrate, permanganate, peroxide, chlorite, chlorate, perchlorate, and persulfate. These may be used singly or in combination of two or more types thereof. Among them, hydrogen peroxide is preferable from the viewpoint of suppressing the contamination of impurity substances.

The mixing amount of the decolorizing agent is preferably 1 mg or more, more preferably 5 mg or more, still more preferably 10 mg or more, yet still more preferably 15 mg or more, and yet still more preferably 20 mg or more with respect to 1 g of the internal olefin sulfonate in the internal olefin sulfonate composition, from the viewpoint of increasing the decolorizing effect (fading rate). The mixing amount is preferably 300 mg or less, more preferably 100 mg or less, still more preferably 40 mg or less, and yet still more preferably 30 mg or less with respect to 1 g of the internal olefin sulfonate in the internal olefin sulfonate composition, from the viewpoint of improving the economic efficiency and suppressing the peroxide value.

In the mixing step (a2), the decolorizing agent may be mixed at one time, or may be mixed in a plurality of portions, and the number of times is not limited.

The decolorizing agent is preferably used as an aqueous solution of the decolorizing agent from the viewpoint of the ease of handling. The concentration of the decolorizing agent in the aqueous solution is not particularly limited, but is preferably 5% or more, more preferably 10% or more, and still more preferably 30% or more, from the viewpoint of reducing the storage tank of the decolorizing agent to improve the economic efficiency. The concentration is preferably 70% or less, more preferably 60% or less, and still more preferably 50% or less, from the viewpoint of the ease of handling.

A temperature in the mixing step (a2) is preferably 40°C or higher, more preferably 60°C or higher, still more preferably 70°C or higher, and yet still more preferably 80°C or higher, from the viewpoint of increasing the decolorizing effect (fading rate) and the viewpoint of reducing the peroxide value. The temperature is preferably 100°C or lower, more preferably 90°C or lower, and still more preferably 85°C or lower, from the viewpoint of the economic efficiency. As described above, the decolorizing reaction is started by mixing the internal olefin sulfonate composition and the decolorizing agent, and thus the temperature in the mixing step (a2) means a temperature at the time of the decolorizing reaction.

The pH of the liquid in the system in the mixing step (a2) is preferably 9.3 or more, and more preferably 9.5 or more, from the viewpoint of suppressing the deterioration of odor and the viewpoint of reducing the peroxide value. The pH is still more preferably 9.8 or more, and yet still more preferably 10.0 or more, from the viewpoint of reducing the peroxide value. The pH is preferably 11.4 or less, and more preferably 11.3 or less, from the viewpoint of suppressing the deterioration of the storage stability. The pH is still more preferably 11.0 or less, yet still more preferably 10.8 or less, and yet still more preferably 10.5 or less, from the viewpoint of increasing the fading rate. As the pH, a value measured by a pH meter installed in a loop type reaction device can be used. The pH is preferably set within an appropriate range in accordance with the properties depending on the type of the alkali compound.

The mixing step (a2) can be performed in a batch, semi-batch, or continuous method, but from the viewpoint of improving the productivity, the mixing step (a2) is preferably performed in a so-called continuous method in which the internal olefin sulfonate composition before decolorizing treatment and the decolorizing agent (aqueous solution of the decolorizing agent) are added while the mixed solution is circulated using the loop type reaction device, and at the same time, the mixed solution subjected to the decolorizing treatment is extracted. In the decolorizing process (A), the concentration of the alkali compound decreases due to the reaction between the internal olefin sulfonate composition and the decolorizing agent, and thus the concentration of the alkali compound is controlled in the adjusting step (a1).

In the mixing step (a2), any type of mixer may be used as long as the mixer can efficiently mix the internal olefin sulfonate composition and the decolorizing agent (aqueous solution of decolorizing agent). Examples of the mixer include an agitating impeller, a stationary type mixer, a collision type mixer, an agitating impeller type mixer, and a vibration type mixer. Examples of the agitating impeller include a small impeller and a large impeller, and examples of the small impeller include a flat paddle impeller, an inclined paddle impeller, and a propeller impeller. Examples of the large impeller include an anchor impeller, a helical ribbon impeller, a screw impeller, Maxblend, a super mix impeller (MR205 (product name, manufactured by Satake MultiMix Corporation) and the like), and Fullzone. Examples of the stationary type mixer include a static mixer manufactured by Noritake Co., Ltd. Examples of the collision type mixer include a high-pressure emulsifying machine manufactured by Nanomizer Inc. Examples of the agitating impeller type mixer include MILDER manufactured by Pacific Machinery & Engineering Co., Ltd., CAVITRON manufactured by Pacific Machinery & Engineering Co., Ltd., and PIPELINE-HOMO MIXER manufactured by PRIMIX Corporation. Among them, from the viewpoint of increasing the agitating efficiency and the fading rate, the agitating impeller or the agitating impeller type mixer is preferable, the large impeller or the agitating impeller type mixer is more preferable, and the agitating impeller type mixer is still more preferable.

When the reaction tank having the agitating impeller or the agitating impeller type mixer is used in the mixing step (a2), the peripheral speed of the agitating impeller is preferably 0.01 m/s or more, more preferably 0.05 m/s or more, still more preferably 0.1 m/s or more, yet still more preferably 0.3 m/s or more, yet still more preferably 0.9 m/s or more, yet still more preferably 4.7 m/s or more, yet still more preferably 7.0 m/s or more, and further yet still more preferably 9.0 m/s or more, from the viewpoint of increasing the decolorizing effect (fading rate). The peripheral speed is preferably 50 m/s or less, more preferably 40 m/s or less, still more preferably 26 m/s or less, and yet still more preferably 15 m/s or less, from the viewpoint of reducing the odor deterioration and improving the economic efficiency. When the mixing step (a2) is performed in a continuous method, the peripheral speed of the agitating impeller is preferably 0.3 m/s or more, more preferably 0.9 m/s or more, still more preferably 4.7 m/s or more, yet still more preferably 7.0 m/s or more, and yet still more preferably 9.0 m/s or more, from the viewpoint of increasing the fading rate. The peripheral speed is preferably 50 m/s or less, more preferably 40 m/s or less, more preferably 26 m/s or less, and still more preferably 15 m/s or less, from the viewpoint of reducing the odor deterioration and improving the economic efficiency. When the mixing step (a2) is performed in a semi-batch method, the peripheral speed of the agitating impeller is preferably 0.01 m/s or more, more preferably 0.1 m/s or more, still more preferably 0.3 m/s or more, and yet still more preferably 0.9 m/s or more, from the viewpoint of increasing the fading rate. The peripheral speed is preferably 50 m/s or less, more preferably 40 m/s or less, still more preferably 26 m/s or less, yet still more preferably 15 m/s or less, and yet still more preferably 10 m/s or less, from the viewpoint of reducing the odor deterioration and improving the economic efficiency. When the mixing step (a2) is performed in a batch method, the peripheral speed of the agitating impeller is preferably 0.01 m/s or more, more preferably 0.1 m/s or more, still more preferably 0.3 m/s or more, yet still more preferably 0.9 m/s or more, and yet still more preferably 1.5 m/s or more, from the viewpoint of increasing the fading rate. The peripheral speed is preferably 50 m/s or less, more preferably 40 m/s or less, still more preferably 26 m/s or less, still more preferably 15 m/s or less, still more preferably 10 m/s or less, still more preferably 5 m/s or less, and still more preferably 3 m/s or less, from the viewpoint of reducing the odor deterioration and improving the economic efficiency.

When the reaction tank having an agitating impeller, or the agitating impeller type mixer is used in the mixing step (a2), a ratio (d/D) of an impeller diameter d to a tank inner diameter D is preferably 0.01 or more, more preferably 0.05 or more, still more preferably 0.1 or more, yet still more preferably 0.3 or more, yet still more preferably 0.4 or more, yet still more preferably 0.5 or more, and yet still more preferably 0.55 or more, from the viewpoint of improving the mixing properties. The ratio (d/D) is preferably less than 1, more preferably 0.9 or less, still more preferably 0.8 or less, and yet still more preferably 0.7 or less, from the viewpoint of improving the economic efficiency. The ratio (d/D) is preferably 0.7 or more, more preferably 0.8 or more, still more preferably 0.9 or more, and preferably less than 1, from the viewpoint of improving the mixing properties.

When the reaction tank having an agitating impeller, or the agitating impeller type mixer is used in the mixing step (a2), a ratio (h/H) of an agitating impeller height h to a tank height H is preferably 0.01 or more, more preferably 0.05 or more, still more preferably 0.1 or more, yet still more preferably 0.3 or more, yet still more preferably 0.4 or more, yet still more preferably 0.5 or more, and yet still more preferably 0.55 or more, from the viewpoint of improving the mixing properties. The ratio (h/H) is preferably less than 1, more preferably 0.9 or less, still more preferably 0.8 or less, and yet still more preferably 0.7 or less, from the viewpoint of suppressing the foaming. The ratio (h/H) is preferably 0.7 or more, more preferably 0.8 or more, still more preferably 0.9 or more, and preferably less than 1, from the viewpoint of improving the mixing properties.

When the reaction tank having agitating impellers or the agitating impeller type mixer is used in the mixing step (a2), the number of stages of the agitating impellers is preferably 1 or more, more preferably 2 or more, and still more preferably 3 or more, from the viewpoint of improving the mixing properties. The number of stages is preferably 10 or less, more preferably 9 or less, and still more preferably 8 or less, from the viewpoint of improving the economic efficiency.

When the adjustment is performed in a batch method using the reaction tank having agitating impellers in the mixing step (a2), the d/D, h/H and the number of stages of the agitating impellers are preferably in the above suitable ranges.

The shear rate in the mixing step (a2) is preferably 5/s or more, more preferably 10/s or more, still more preferably 100/s or more, yet still more preferably 500/s or more, yet still more preferably 1,000/s or more, and yet still more preferably 1,500/s or more, from the viewpoint of increasing the fading rate. The shear rate is preferably 100,000/s or less, more preferably 80,000/s or less, still more preferably 70,000/s or less, yet still more preferably 50,000/s or less, yet still more preferably 40,000/s or less, yet still more preferably 30,000/s or less, and yet still more preferably 20,000/s or less, from the viewpoint of reducing the odor deterioration and improving the economic efficiency. When the mixing step (a2) is performed in a continuous method, the shear rate is preferably 100/s or more, more preferably 500/s or more, still more preferably 1,000/s or more, and still more preferably 1,500/s or more, from the viewpoint of increasing the fading rate. The shear rate is preferably 100,000/s or less, more preferably 80,000/s or less, still more preferably 70,000/s or less, yet still more preferably 50,000/s or less, yet still more preferably 40,000/s or less, yet still more preferably 30,000/s or less, and yet still more preferably 20,000/s or less, from the viewpoint of reducing the odor deterioration and improving the economic efficiency. When the mixing step (a2) is performed in a semi-batch method, the shear rate is preferably 10/s or more, more preferably 100/s or more, still more preferably 500/s or more, yet still more preferably 1,000/s or more, and yet still more preferably 1,500/s or more, from the viewpoint of increasing the fading rate. The shear rate is preferably 100,000/s or less, more preferably 80,000/s or less, still more preferably 70,000/s or less, yet still more preferably 50,000/s or less, yet still more preferably 40,000/s or less, yet still more preferably 30,000/s or less, and yet still more preferably 20,000/s or less, from the viewpoint of reducing the odor deterioration and improving the economic efficiency. When the mixing step (a2) is performed in a batch method, the shear rate is preferably 5/s or more, more preferably 8/s or more, still more preferably 10/s or more, and yet still more preferably 12/s or more, from the viewpoint of increasing the fading rate. The shear rate is preferably 5,000/s or less, more preferably 1,000/s or less, still more preferably 500/s or less, yet still more preferably 100/s or less, and yet still more preferably 30/s or less, from the viewpoint of reducing the odor deterioration and improving the economic efficiency.

Stirring power per unit liquid amount (Pv) of the mixer used in the mixing step (a2) is preferably 0.1 kW/m$^3$ or more, more preferably 0.2 kW/m$^3$ or more, and still more preferably 0.3 kW/m$^3$ or more, from the viewpoint of increasing the fading rate. The power is preferably 100 kW/m$^3$ or less, more preferably 50 kW/m$^3$ or less, still more preferably 30 kW/m$^3$ or less, yet still more preferably 10 kW/m$^3$ or less, yet still more preferably 5 kW/m$^3$ or less, and yet still more preferably 1 kW/m$^3$ or less, from the viewpoint of reducing the odor deterioration and improving the economic efficiency.

The pressure in the system in the mixing step (a2) is preferably 0 MPaG or more, more preferably 0.01 MPaG or more, still more preferably 0.05 MPaG or more, yet still more preferably 0.1 MPaG or more, and yet still more preferably 0.2 MPaG or more, from the viewpoint of reducing the device volume and the peroxide value. The pressure is preferably 10 MPaG or less, more preferably 5 MPaG or less, still more preferably 1 MPaG or less, and still more preferably 0.5 MPaG or less, from the viewpoint of improving the economic efficiency. Here, G of MPaG indicates a gauge pressure.

The pressure in the system in the adjusting step when the adjusting step (a1) is performed after the mixing step (a2) or when the adjusting step (a1) is performed simultaneously with the mixing step (a2) is preferably 0 MPaG or more, more preferably 0.01 MPaG or more, still more preferably 0.05 MPaG or more, yet still more preferably 0.1 MPaG or more, and yet still more preferably 0.2 MPaG or more, from the viewpoint of the ease of controlling the concentration of the alkali compound. The pressure is preferably 10 MPaG or less, more preferably 5 MPaG or less, still more preferably 1 MPaG or less, and yet still more preferably 0.5 MPaG or less, from the viewpoint of improving the economic efficiency.

Although the decolorizing process (A) includes the mixing step (a2) and the adjusting step (a1), the decolorizing process (A) may include other steps. For example, the decolorizing process (A) preferably includes an aging step (b).

[Aging step (b)]

The aging step (b) is a step of maintaining a state where the decolorizing agent and the internal olefin sulfonate composition coexist without newly adding the decolorizing agent after the mixing step (a2), and it is not necessary to mix them by a mixer or the like. In the present invention, it is preferable to perform the aging step (b) from the viewpoint of reducing the peroxide value.

The suitable temperature range of the aging step (b) may be the same as the suitable temperature range of the mixing step (a2) from the viewpoint of the economic efficiency, but is, for example, preferably 40°C or higher, more preferably 50°C or higher, and still more preferably 60°C or higher, from the viewpoint of the economic efficiency and the reduction of the peroxide value. The suitable temperature range is preferably 120°C or lower, more preferably 110°C or lower, and still more preferably 100°C or lower, from the viewpoint of improving the economic efficiency.

The reaction device for performing the aging step (b) is not particularly limited, and in the case of the batch method, a thermostatic bath or a tank type reactor or the like can be used. In the case of the semi-batch method or the continuous method, a tank type reactor can be used. Examples of the tank shape of the tank type reactor include a cylindrical shape, a square shape, and a spherical shape, and the tank type reactor is preferably a cylindrical reactor from the viewpoint of improving the economic efficiency. Examples of the tank type reactor having a cylindrical tank include a horizontal cylindrical reactor and a vertical cylindrical reactor, and a vertical cylindrical reactor is preferable from the viewpoint of improving the reaction efficiency. In order to improve the reaction efficiency, a dispersion mechanism such as a porous plate or a dispersion plate may be provided in the reactor as necessary.

When the aging step (b) is performed using the vertical cylindrical reactor, a ratio ($H_1/D_1$) of the height $H_1$ of the vertical cylindrical reactor to the inner diameter $D_1$ of the reactor is preferably 0.1 or more, more preferably 1 or more, and still more preferably 1.5 or more, from the viewpoint of improving the reaction efficiency. The ratio ($H_1/D_1$) is preferably 300 or less, more preferably 200 or less, still more preferably 100 or less, yet still more preferably 50 or less, yet still more preferably 30 or less, yet still more preferably 10 or less, and yet still more preferably 5 or less, from the viewpoint of suppressing the pressure loss and improving the economic efficiency.

A residence time in the aging step (b) is preferably 10 minutes or more, more preferably 20 minutes or more, still more preferably 30 minutes or more, yet still more preferably 45 minutes or more, and yet still more preferably 60 minutes or more, from the viewpoint of reducing the peroxide value. The residence time is preferably 500 minutes or less, more preferably 400 minutes or less, still more preferably 300 minutes or less, and yet still more preferably 200 minutes or less, from the viewpoint of improving the economic efficiency.

The pressure in the aging step (b) is preferably 0 MPaG or more, more preferably 0.01 MPaG or more, more preferably 0.05 MPaG or more, still more preferably 0.1 MPaG or more, and yet still more preferably 0.2 MPaG or more, from the viewpoint of reducing the device volume and the peroxide value. The pressure is preferably 10 MPaG or less, more preferably 5 MPaG or less, still more preferably 1 MPaG or less, and yet still more preferably 0.5 MPaG or less, from the viewpoint of improving the economic efficiency.

The aging step (b) may be performed in a continuous method, a semi-batch method, or a batch method, but is preferably performed in a batch method or a semi-batch method from the viewpoint of the reaction efficiency, and is preferably performed in a continuous method from the viewpoint of improving the productivity.

When the aging step (b) is performed in a continuous method, the linear velocity of the internal olefin sulfonate composition is preferably 0.001 mm/s or more, more preferably 0.005 mm/s or more, still more preferably 0.01 mm/s or more, and yet still more preferably 0.1 or more, from the viewpoint of maintaining the uniformity of the composition of the internal olefin sulfonate composition. The linear velocity is preferably 1,000 mm/s or less, more preferably 800 mm/s or less, still more preferably 500 mm/s or less, yet still more preferably 300 mm/s or less, yet still more preferably 100 mm/s or less, yet still more preferably 50 mm/s or less, yet still more preferably 10 mm/s or less, yet still more preferably 5 mm/s or less, and yet still more preferably 1 mm/s or less, from the viewpoint of reducing the peroxide value.

The fading rate of the decolorized internal olefin sulfonate composition obtained in the decolorizing process (A) with respect to the internal olefin sulfonate composition before decolorizing is preferably 50% or more, more preferably 60% or more, more preferably 63% or more, and still more preferably 650 or more. Here, the fading rate can be measured by a method described in Examples.

The peroxide value of the decolorized internal olefin sulfonate composition obtained in the decolorizing process (A) is preferably 37.0 meq or less, more preferably 30.0 meq or less, more preferably 20.0 meq or less, more preferably 10 meq or less, still more preferably 7 meq or less, and still more preferably 5 meq or less with respect to 1 kg of the internal olefin sulfonate in the internal olefin sulfonate composition, from the viewpoint of the stability of the decolorized internal olefin sulfonate composition. The peroxide value can also be set to the above concentration by subjecting the decolorizing agent such as the oxidizing agent remaining to a reduction treatment to decompose the decolorizing agent. Here, the peroxide value can be measured by a method described in Examples.

The decolorized internal olefin sulfonate composition obtained by the production method of the present invention can be used for various applications as it is, but may be further subjected to a defoaming step or a pH adjusting step, or may be subjected to purification such as desalting. An effective component concentration may be adjusted by an operation such as water addition or concentration, or the decolorized internal olefin sulfonate composition may be used by being mixed with other surfactants or solvents.

Each of the steps (adjusting step, mixing step, aging step) may be combined with the defoaming step.

\<pH adjusting step\>

After the decolorizing process (A), the pH adjusting step of adjusting the pH of the internal olefin sulfonate composition after decolorizing treatment may be performed.

When the pH adjusting step is performed, the pH of the internal olefin sulfonate composition after pH adjustment is preferably 8.5 or more, and more preferably 9.0 or more, from the viewpoint of suppressing the increase of the amount of impurities in the decolorized internal olefin sulfonate composition. The pH is preferably 12.0 or less, more preferably 11.4 or less, still more preferably 11.0 or less, and yet still more preferably 10.5 or less, from the viewpoint of the ease of handling. Here, the pH is a pH of 25°C measured by adjusting the concentration of the internal olefin sulfonate in the internal olefin sulfonate composition after decolorizing treatment to 10% by mass.

An acid used for pH adjustment is not particularly limited, and examples thereof include inorganic acids such as sulfuric acid, hydrochloric acid, phosphoric acid, and boric acid; and organic acids such as citric acid, malic acid, maleic acid, fumaric acid, and succinic acid. These may be used singly or in combination of two or more types thereof. Among them, one or more selected from the group consisting of citric acid and phosphoric acid are preferable, and citric acid is more preferable. In order to improve the pH controllability, one or more kinds of pH buffering agents may be used or used in combination. The pH buffering agent is not particularly limited, and examples thereof include acetic acid, citric acid, phosphoric acid, EDTA, and sodium carbonate.

The acid is preferably used as an aqueous acid solution from the viewpoint of the ease of handling.

The decolorized internal olefin sulfonate composition obtained by the production method of the present invention has a good color fading effect, low odor, and good storage stability, and thus can be suitably used for various applications such as body detergents, shampoos, clothing detergents, and tableware detergents.

The present invention and preferred embodiments of the present invention are described below.

\<1\> A method for producing a decolorized internal olefin sulfonate composition comprising a decolorizing process (A) of performing an adjusting step (a1) of adjusting a concentration of an alkali compound in a system in which an internal olefin sulfonate composition containing an internal olefin sulfonate is present and a mixing step (a2) of mixing a decolorizing agent and the internal olefin sulfonate composition present in the system,
wherein the adjusting step (a1) is a step of adjusting the concentration of the alkali compound in the decolorizing process (A) so that the concentration of the alkali compound after mixing the decolorizing agent and the internal olefin sulfonate composition present in the system is 0.004 mmol or more and 0.26 mmol or less with respect to 1 g of the internal olefin sulfonate.

\<2\> The method for producing a decolorized internal olefin sulfonate composition according to \<1\>, wherein the adjusting step (a1) is performed after the mixing step (a2) is performed.

<3> The method for producing a decolorized internal olefin sulfonate composition according to <1>, wherein the mixing step (a2) is performed after the adjusting step (a1) is performed.

<4> The method for producing a decolorized internal olefin sulfonate composition according to <1>, wherein the adjusting step (a1) and the mixing step (a2) are performed in parallel.

<5> The method for producing a decolorized internal olefin sulfonate composition according to any one of <1> to <4>, wherein the decolorizing process (A) is performed after the neutralization process or after the hydrolysis process, and is preferably performed after the hydrolysis process.

<6> The method for producing a decolorized internal olefin sulfonate composition according to any one of <1> to <5>, wherein the concentration of the alkali compound in the liquid in the system in which the concentration of the alkali compound is adjusted in the adjusting step (a1) is preferably 0.005 mmol or more, more preferably 0.010 mmol or more, still more preferably 0.015 mmol or more, yet still more preferably 0.020 mmol or more, and yet still more preferably 0.030 mmol or more, with respect to 1 g of the internal olefin sulfonate, and is preferably 0.23 mmol or less, more preferably 0.215 mmol or less, still more preferably 0.20 mmol or less, yet still more preferably 0.15 mmol or less, yet still more preferably 0.10 mmol or less, yet still more preferably 0.08 mmol or less, and yet still more preferably 0.06 mmol or less.

<7> The method for producing a decolorized internal olefin sulfonate composition according to any one of <1> to <5>, wherein the concentration of the alkali compound in the liquid in the system in which the concentration of the alkali compound is adjusted in the adjusting step (a1) is preferably 0.010 mmol or more, more preferably 0.015 mmol or more, still more preferably 0.020 mmol or more, yet still more preferably 0.025 mmol or more, yet still more preferably 0.030 mmol or more, and yet still more preferably 0.035 mmol or more, with respect to 1 g of the internal olefin sulfonate, and is preferably 0.270 mmol or less, more preferably 0.240 mmol or less, still more preferably 0.210 mmol or less, yet still more preferably 0.240 mmol or less, yet still more preferably 0.220 mmol or less, yet still more preferably 0.210 mmol or less, yet still more preferably 0.20 mmol or less, yet still more preferably 0.18 mmol or less, yet still more preferably 0.16 mmol or less, yet still more preferably 0.10 mmol or less and yet still more preferably 0.08 mmol or less.

<8> The method for producing a decolorized internal olefin sulfonate composition according to any one of <1> to <7>, wherein the adjusting method is performed by extracting the liquid in the system and analyzing the concentration of the alkali compound in the extracted liquid promptly at room temperature.

<9> The method for producing a decolorized internal olefin sulfonate composition according to any one of <1> to <8>, wherein the alkali compound is preferably an inorganic alkali compound, more preferably an alkali metal hydroxide, still more preferably at least one selected from the group consisting of sodium hydroxide and potassium hydroxide.

<10> The method for producing a decolorized internal olefin sulfonate composition according to any one of <1> to <9>, wherein the adjusting step (a1) is preferably performed in a batch, semi-batch, or continuous method, and more preferably performed in a continuous method.

<11> The method for producing a decolorized internal olefin sulfonate composition according to any one of <1> to <10>, wherein adjusting step (a1) is performed using the loop type reaction device.

<12> The method for producing a decolorized internal olefin sulfonate composition according to any one of <1> to <11>, wherein the adjusting step (a1) is performed in a continuous method with a circulation system.

<13> The method for producing a decolorized internal olefin sulfonate composition according to <12>, wherein a circulation ratio of the circulation system is preferably greater than 0 times, more preferably 1 time or more, more preferably 2 times or more, still more preferably 3 times or more, and still more preferably 4 times or more, and is preferably 20 times or less, more preferably 10 times or less, and still more preferably 8 times or less.

<14> The method for producing a decolorized internal olefin sulfonate composition according to any one of <1> to <13>, wherein a reaction tank having an agitating impeller or an agitating impeller type mixer is used in the adjusting step (a1), and a peripheral speed of the agitating impeller is 0.01 m/s or more and 50 m/s or less.

<15> The method for producing a decolorized internal olefin sulfonate composition according to <14>, wherein a peripheral speed of the agitating impeller in the adjusting step (a1) is preferably 0.01 m/s or more, more preferably 0.05 m/s or more, still more preferably 0.1 m/s or more, yet still more preferably 0.3 m/s or more, yet still more preferably 0.9 m/s or more, yet still more preferably 4.7 m/s or more, yet still more preferably 7.0 m/s or more, and yet still more preferably 9.0 m/s or more, and is preferably 50 m/s or less, more preferably 40 m/s or less, still more preferably 26 m/s or less, and yet still more preferably 15 m/s or less.

<16> The method for producing a decolorized internal olefin sulfonate composition according to <14>, wherein the adjusting step (a1) is performed in a continuous method, and a peripheral speed of the agitating impeller is preferably 0.1 m/s or more, more preferably 0.3 m/s or more, still more preferably 4 m/s or more, yet still more preferably 7.0 m/s or more, and yet still more preferably 9.0 m/s or more, and is preferably 50 m/s or less, more preferably 40 m/s or less, more preferably 26 m/s or less, and still more preferably 15 m/s or less.

<17> The method for producing a decolorized internal olefin sulfonate composition according to <14>, wherein the adjusting step (a1) is performed in a semi-batch method, and a peripheral speed of the agitating impeller is preferably 0.1 m/s or more, more preferably 0.3 m/s or more, still more preferably 0.5 m/s or more, and yet still more preferably 0.7 m/s or more, and is preferably 50 m/s or less, more preferably 40 m/s or less, still more preferably 26 m/s or less, yet still more preferably 15 m/s or less, yet still more preferably 10 m/s or less, and yet still more preferably 8 m/s or less.

<18> The method for producing a decolorized internal olefin sulfonate composition according to <14>, wherein the adjusting step (a1) is performed in a batch method, a peripheral speed of the agitating impeller is preferably 0.1 m/s or more, more preferably 0.3 m/s or more, still more preferably 0.9 m/s or more, yet still more preferably 1.0 m/s or more, and yet still more preferably 1.5 m/s or more, and is preferably 50 m/s or less, more preferably 40 m/s or less, still more preferably 26 m/s or less, yet still more preferably 15 m/s or less, yet still more preferably 10 m/s or less, yet still more preferably 5 m/s or less, and yet still more preferably 3 m/s or less.

<19> The method for producing a decolorized internal olefin sulfonate composition according to any one of <1> to <18>, wherein a reaction tank having an agitating impeller or an agitating impeller type mixer is used in the adjusting step (a1), and a ratio (d/D) of an impeller diameter d to a tank inner diameter D is preferably 0.01 or more, more preferably 0.05 or more, still more preferably 0.1 or more, yet still more preferably 0.3 or more, yet still more preferably 0.4 or more, yet still more preferably 0.5 or more, and yet still more preferably 0.55 or more, and is preferably less than 1, more preferably 0.9 or less, still more preferably 0.8 or less, and yet still more preferably 0.7 or less, and is preferably 0.7 or more, more preferably 0.8 or more, still more preferably 0.9 or more, and preferably less than 1 from the viewpoint of improving the mixing properties.

<20> The method for producing a decolorized internal olefin sulfonate composition according to any one of <1> to <19>, wherein a reaction tank having an agitating impeller or an agitating impeller type mixer is used in the adjusting step (a1), and a ratio (h/H) of an agitating impeller height h to a tank height H is preferably 0.01 or more, more preferably 0.05 or more, still more preferably 0.1 or more, yet still more preferably 0.3 or more, yet still more preferably 0.4 or more, yet still more preferably 0.5 or more, and yet still more preferably 0.55 or more, and is preferably less than 1, more preferably 0.9 or less, still more preferably 0.8 or less, and yet still more preferably 0.7 or less, and is preferably 0.7 or more, more preferably 0.8 or more, still more preferably 0.9 or more, and preferably less than 1 from the viewpoint of improving the mixing properties.

<21> The method for producing a decolorized internal olefin sulfonate composition according to any one of <1> to <20>, wherein a reaction tank having an agitating impeller or an agitating impeller type mixer is used in the adjusting step (a1), and the number of stages of the agitating impellers is preferably 1 or more, more preferably 2 or more, and still more preferably 3 or more, and is preferably 10 or less, more preferably 9 or less, and still more preferably 8 or less.

<22> The method for producing a decolorized internal olefin sulfonate composition according to any one of <1> to <21>, wherein the adjusting step (a1) is performed under shear conditions.

<23> The method for producing a decolorized internal olefin sulfonate composition according to <22>, wherein a shear rate is preferably 5/s or more, more preferably 10/s or more, still more preferably 100/s or more, yet still more preferably 500/s or more, yet still more preferably 1,000/s or more, and yet still more preferably 1,500/s or more, and is preferably 100,000/s or less, more preferably 50,000/s or less, still more preferably 30,000/s or less, and yet still more preferably 20,000/s or less.

<24> The method for producing a decolorized internal olefin sulfonate composition according to <22>, wherein the adjusting step (a1) is performed in a continuous method, and a shear rate is preferably 100/s or more, more preferably 500/s or more, still more preferably 1,000/s or more, and still more preferably 1,500/s or more, and is preferably 100,000/s or less, more preferably 50,000/s or less, still more preferably 10,000/s or less, and yet still more preferably 8,000/s or less.

<25> The method for producing a decolorized internal olefin sulfonate composition according to <22>, wherein the adjusting step (a1) is performed in a semi-batch method, and a shear rate is preferably 10/s or more, more preferably 100/s or more, still more preferably 500/s or more, yet still more preferably 1,000/s or more, and yet still more preferably 1,500/s or more, and is preferably 100,000/s or less, more preferably 50,000/s or less, still more preferably 40,000/s or less, yet still more preferably 30,000/s or less, yet still more preferably 20,000/s or less, yet still more preferably 10,000/s or less, and yet still more preferably 8,000/s or less.

<26> The method for producing a decolorized internal olefin sulfonate composition according to <22>, wherein the adjusting step (a1) is performed in a batch method, and a shear rate is preferably 5/s or more, more preferably 8/s or more, still more preferably 10/s or more, and yet still more preferably 12/s or more, and is preferably 5,000/s or less, more preferably 1,000/s or less, still more preferably 500/s or less, yet still more preferably 100/s or less, and yet still more preferably 50/s or less.

<27> The method for producing a decolorized internal olefin sulfonate composition according to any one of <1> to <26>, wherein stirring power per unit liquid amount (Pv) of the mixer used in the adjusting step (a1) is preferably 0.1 $kW/m^3$ or more, more preferably 0.2 $kW/m^3$ or more, and still more preferably 0.3 $kW/m^3$ or more, and is preferably 100 $kW/m^3$ or less, more preferably 50 $kW/m^3$ or less, still more preferably 30 $kW/m^3$ or less, yet still more preferably 10 $kW/m^3$ or less, yet still more preferably 5 $kW/m^3$ or less, and yet still more preferably 1 $kW/m^3$ or less.

<28> The method for producing a decolorized internal olefin sulfonate composition according to any one of <1> to <27>, wherein a pressure in the system in the adjusting step (a1) is preferably 0 MPaG or more, more preferably 0.01 MPaG or more, more preferably 0.05 MPaG or more, still more preferably 0.1 MPaG or more, and still more preferably 0.2 MPaG or more, and is preferably 10 MPaG or less, more preferably 5 MPaG or less, still more preferably 1 MPaG or less, and yet still more preferably 0.5 MPaG or less.

<29> The method for producing a decolorized internal olefin sulfonate composition according to any one of <1> to <28>, wherein the decolorizing agent is an oxidizing agent.

<30> The method for producing a decolorized internal olefin sulfonate composition according to any one of <1> to <29>, wherein the decolorizing agent is preferably at least one selected from the group consisting of hydrogen peroxide, hypochlorous acid, nitric acid, potassium nitrate, permanganate, peroxide, chlorite, chlorate, perchlorate, and persulfate, and more preferably hydrogen peroxide.

<31> The method for producing a decolorized internal olefin sulfonate composition according to any one of <1> to <30>, wherein a mixing amount of the decolorizing agent is preferably 1 mg or more, more preferably 5 mg or more, still more preferably 10 mg or more, yet still more preferably 15 mg or more, and yet still more preferably 20 mg or more, and is preferably 300 mg or less, more preferably 100 mg or less, still more preferably 40 mg or less, and yet still more preferably 30 mg or less, with respect to 1 g of the internal olefin sulfonate in the internal olefin sulfonate composition.

<32> The method for producing a decolorized internal olefin sulfonate composition according to any one of <1> to <31>, wherein a mixing amount of the decolorizing agent is preferably 1 mg or more and 300 mg or less, more preferably 5 mg or more and 100 mg or less, still more preferably 10 mg or more and 40 mg or less, yet still more preferably 15 mg or more and 30 mg or less, and yet still more preferably 20 mg or more and 30 mg or less, with respect to 1 g of the internal olefin sulfonate in the internal olefin sulfonate composition.

<33> The method for producing a decolorized internal olefin sulfonate composition according to any one of <1> to <32>, wherein the decolorizing agent is an aqueous solution of the decolorizing agent, and a concentration of the decolorizing agent in the aqueous solution is preferably 5% or more, more preferably 10% or more, and still more preferably 30% or more, and is preferably 70% or less, more preferably 60% or less, and still more preferably 50% or less.

<34> The method for producing a decolorized internal olefin sulfonate composition according to any one of <1> to <33>, wherein a temperature in the mixing step (a2) is preferably 40°C or higher, more preferably 60°C or higher, still more preferably 70°C or higher, and yet still more preferably 80°C or higher, and is preferably 100°C or lower, more preferably 90°C or lower, and still more preferably 85°C or lower.

<35> The method for producing a decolorized internal olefin sulfonate composition according to any one of <1> to <34>, wherein a temperature in the mixing step (a2) is preferably 40°C or higher and 100°C or lower, more preferably 60°C or higher and 90°C or lower, still more preferably 70°C or higher and 85°C or lower, and yet still more preferably 80°C or higher and 85°C or lower.

<36> The method for producing a decolorized internal olefin sulfonate composition according to any one of <1> to <35>, wherein a pH of the liquid in the system in the mixing step (a2) is preferably 9.3 or more, more preferably 9.5 or more, still more preferably 9.8 or more, yet still more preferably 10.0 or more, yet still more preferably 10.2 or more, and yet still more preferably 10.4 or more, and is preferably 11.4 or less, more preferably 11.3 or less, still more preferably 11.0 or less, yet still more preferably 10.8 or less, and yet still more preferably 10.5 or less.

<37> The method for producing a decolorized internal olefin sulfonate composition according to any one of <1> to <36>, wherein the mixing step (a2) is preferably performed in a batch, semi-batch, or continuous method, and is more preferably performed in a continuous method.

<38> The method for producing a decolorized internal olefin sulfonate composition according to any one of <1> to <37>, wherein the mixing step (a2) is performed in a continuous method in which the internal olefin sulfonate composition before decolorizing treatment and the decolorizing agent (aqueous solution of the decolorizing agent) are added while the mixed solution is circulated using the loop type reaction device, and at the same time, the mixed solution subjected to the decolorizing treatment is extracted.

<39> The method for producing a decolorized internal olefin sulfonate composition according to any one of <1> to <38>, wherein the mixing step (a2) is performed using a reaction tank having an agitating impeller or an agitating impeller type mixer, and a peripheral speed of the agitating impeller is 0.01 m/s or more and 50 m/s or less.

<40> The method for producing a decolorized internal olefin sulfonate composition according to <39>, wherein in the mixing step (a2), a peripheral speed of the agitating impeller is preferably 0.01 m/s or more, more preferably 0.05 m/s or more, still more preferably 0.1 m/s or more, yet still more preferably 0.3 m/s or more, yet still more preferably 0.9 m/s or more, yet still more preferably 4.7 m/s or more, yet still more preferably 7.0 m/s or more, and further yet still more preferably 9.0 m/s or more, and is preferably 50 m/s or less, more preferably 40 m/s or less, still more preferably 26 m/s or less, and yet still more preferably 15 m/s or less.

<41> The method for producing a decolorized internal olefin sulfonate composition according to <39>, wherein the mixing step (a2) is performed in a continuous method, and a peripheral speed of the agitating impeller is preferably 0.3 m/s or more, more preferably 0.9 m/s or more, still more preferably 4.7 m/s or more, yet still more preferably 7.0 m/s or more, and yet still more preferably 9.0 m/s or more, and is preferably 50 m/s or less, more preferably 40 m/s or less, more preferably 26 m/s or less, and still more preferably 15 m/s or less.

<42> The method for producing a decolorized internal olefin sulfonate composition according to <39>, wherein the mixing step (a2) is performed in a semi-batch method, and a peripheral speed of the agitating impeller is preferably 0.01 m/s or more, more preferably 0.1 m/s or more, still more preferably 0.3 m/s or more, and yet still more preferably 0.9 m/s or more, and is preferably 50 m/s or less, more preferably 40 m/s or less, still more preferably 26 m/s or less, yet still more preferably 15 m/s or less, and yet still more preferably 10 m/s or less.

<43> The method for producing a decolorized internal olefin sulfonate composition according to <39>, wherein the mixing step (a2) is performed in a batch method, and a peripheral speed of the agitating impeller is preferably 0.01 m/s or more, more preferably 0.1 m/s or more, still more preferably 0.3 m/s or more, yet still more preferably 0.9 m/s or more, and yet still more preferably 1.5 m/s or more, and is preferably 50 m/s or less, more preferably 40 m/s or less, still more preferably 26 m/s or less, yet still more preferably 15 m/s or less, yet still more preferably 10 m/s or less, yet still more preferably 5 m/s or less, and yet still more preferably 3 m/s or less.

<44> The method for producing a decolorized internal olefin sulfonate composition according to any one of <1> to <43>, wherein the mixing step (a2) is performed using a reaction tank having an agitating impeller or an agitating impeller type mixer, and a ratio (d/D) of an impeller diameter d to a tank inner diameter D is preferably 0.01 or more, more preferably 0.05 or more, still more preferably 0.1 or more, yet still more preferably 0.3 or more, yet still more preferably 0.4 or more, yet still more preferably 0.5 or more, and yet still more preferably 0.55 or more, and is preferably less than 1, more preferably 0.9 or less, still more preferably 0.8 or less, and yet still more preferably 0.7 or less, and is preferably 0.7 or more, more preferably 0.8 or more, still more preferably 0.9 or more, and preferably less than 1 from the viewpoint of improving the mixing properties.

<45> The method for producing a decolorized internal olefin sulfonate composition according to any one of <1> to <44>, wherein the mixing step (a2) is performed using a reaction tank having an agitating impeller or an agitating impeller type mixer, and a ratio (h/H) of an agitating impeller height h to a tank height H is preferably 0.01 or more, more preferably 0.05 or more, still more preferably 0.1 or more, yet still more preferably 0.3 or more, yet still more preferably 0.4 or more, yet still more preferably 0.5 or more, and yet still more preferably 0.55 or more, and is preferably less than 1, more preferably 0.9 or less, still more preferably 0.8 or less, and yet still more preferably 0.7 or less, and is preferably 0.7 or more, more preferably 0.8 or more, still more preferably 0.9 or more, and preferably less than 1 from the viewpoint of improving the mixing properties.

<46> The method for producing a decolorized internal olefin sulfonate composition according to any one of <1> to <45>, wherein the mixing step (a2) is performed using a reaction tank having an agitating impeller or an agitating impeller type mixer, and the number of stages of the agitating impellers is preferably 1 or more, more preferably 2 or more, and still more preferably 3 or more, and is preferably 10 or less, more preferably 9 or less, and still more preferably 8 or less.

<47> The method for producing a decolorized internal olefin sulfonate composition according to any one of <1> to <46>, wherein a shear rate in the mixing step (a2) is preferably 5/s or more, more preferably 10/s or more, still more preferably 100/s or more, yet still more preferably 500/s or more, yet still more preferably 1,000/s or more, and yet still more preferably 1,500/s or more, and is preferably 100,000/s or less, more preferably 80,000/s or less, still more preferably 70,000/s or less, yet still more preferably 50,000/s or less, yet still more preferably 40,000/s or less, yet still more preferably 30,000/s or less, and yet still more preferably 20,000/s or less.

<48> The method for producing a decolorized internal olefin sulfonate composition according to any one of <1> to <46>, wherein the mixing step (a2) is performed in a continuous method, and a shear rate in the mixing step (a2) is preferably 100/s or more, more preferably 500/s or more, still more preferably 1,000/s or more, and yet still more preferably 1,500/s or more, and is preferably 100,000/s or less, more preferably 80,000/s or less, still more preferably 70,000/s or less, yet still more preferably 50,000/s or less, yet still more preferably 40,000/s or less, yet still more preferably 30,000/s or less, and yet still more preferably 20,000/s or less.

<49> The method for producing a decolorized internal olefin sulfonate composition according to any one of <1> to <46>, wherein the mixing step (a2) is performed in a semi-batch method, and a shear rate in the mixing step (a2) is preferably 10/s or more, more preferably 100/s or more, still more preferably 500/s or more, yet still more preferably 1,000/s or more, and yet still more preferably 1,500/s or more, and is preferably 100,000/s or less, more preferably 80,000/s or less, still more preferably 70,000/s or less, yet still more preferably 50,000/s or less, yet still more preferably 40,000/s or less, yet still more preferably 30,000/s or less, and yet still more preferably 20,000/s or less.

<50> The method for producing a decolorized internal olefin sulfonate composition according to any one of <1> to <46>, wherein the mixing step (a2) is performed in a batch method, and a shear rate in the mixing step (a2) is preferably 5/s or more, more preferably 8/s or more, still more preferably 10/s or more, and yet still more preferably 12/s or more, and is preferably 5,000/s or less, more preferably 1,000/s or less, still more preferably 500/s or less, yet still more preferably 100/s or less, and yet still more preferably 30/s or less.

<51> The method for producing a decolorized internal olefin sulfonate composition according to any one of <1> to <50>, wherein stirring power per unit liquid amount (Pv) of the mixer used in the mixing step (a2) is preferably 0.1 $kW/m^3$ or more, more preferably 0.2 $kW/m^3$ or more, and still more preferably 0.3 $kW/m^3$ or more, and is preferably 100 $kW/m^3$ or less, more preferably 50 $kW/m^3$ or less, still more preferably 30 $kW/m^3$ or less, yet still more preferably 10 $kW/m^3$ or less, yet still more preferably 5 $kW/m^3$ or less, and yet still more preferably 1 $kW/m^3$ or less.

<52> The method for producing a decolorized internal olefin sulfonate composition according to any one of <1> to <51>, wherein a pressure in the system in the mixing step (a2) is preferably 0 MPaG or more, more preferably 0.01 MPaG or more, still more preferably 0.05 MPaG or more, yet still more preferably 0.1 MPaG or more, and yet still more preferably 0.2 MPaG or more, and is preferably 10 MPaG or less, more preferably 5 MPaG or less, still more preferably 1 MPaG or less, and still more preferably 0.5 MPaG or less.

<53> The method for producing a decolorized internal olefin sulfonate composition according to any one of <1> to <52>, wherein a pressure in the system in the adjusting step when the adjusting step (a1) is performed after the mixing step (a2) or when the adjusting step (a1) is performed simultaneously with the mixing step (a2) is preferably 0 MPaG or more, more preferably 0.01 MPaG or more, still more preferably 0.05 MPaG or more, yet still more preferably 0.1 MPaG or more, and yet still more preferably 0.2 MPaG or more, and is preferably 10 MPaG or less, more preferably 5 MPaG or less, still more preferably 1 MPaG or less, and yet still more preferably 0.5 MPaG or less.

<54> The method for producing a decolorized internal olefin sulfonate composition according to any one of <1> to <53>, further containing an aging step (b) after the mixing step (a2).

<55> The method for producing a decolorized internal olefin sulfonate composition according to <54>, wherein a temperature range of the aging step (b) is preferably 40°C or higher, more preferably 50°C or higher, and still more preferably 60°C or higher, and is preferably 120°C or lower, more preferably 110°C or lower, and still more preferably 100°C or lower.

<56> The method for producing a decolorized internal olefin sulfonate composition according to <54> or <55>, wherein the aging step (b) is performed using the vertical cylindrical reactor, and a ratio ($H_1/D_1$) of the height $H_1$ of the vertical cylindrical reactor to the inner diameter $D_1$ of the reactor is preferably 0.1 or more, more preferably 1 or more, and still more preferably 1.5 or more, and is preferably 300 or less, more preferably 200 or less, still more preferably 100 or less, yet still more preferably 50 or less, yet still more preferably 30 or less, yet still more preferably 10 or less, and yet still more preferably 5 or less.

<57> The method for producing a decolorized internal olefin sulfonate composition according to any one of <54> to <56>, wherein a residence time in the aging step (b) is preferably 10 minutes or more, more preferably 20 minutes or more, still more preferably 30 minutes or more, yet still more preferably 45 minutes or more, and yet still more preferably 60 minutes or more, and is preferably 500 minutes or less, more preferably 400 minutes or less, still more preferably 300 minutes or less, and yet still more preferably 200 minutes or less.

<58> The method for producing a decolorized internal olefin sulfonate composition according to any one of <54> to <57>, wherein a pressure in the aging step (b) is preferably 0 MPaG or more, more preferably 0.01 MPaG or more, more preferably 0.05 MPaG or more, still more preferably 0.1 MPaG or more, and yet still more preferably 0.2 MPaG or more, and is preferably 10 MPaG or less, more preferably 5 MPaG or less, still more preferably 1 MPaG or less, and yet still more preferably 0.5 MPaG or less.

<59> The method for producing a decolorized internal olefin sulfonate composition according to any one of <54> to <58>, wherein the aging step (b) is preferably performed in a continuous method, a semi-batch method, or a batch method, and is more preferably performed in a batch method or a semi-batch method.

<60> The method for producing a decolorized internal olefin sulfonate composition according to any one of <54> to <59>, wherein the aging step (b) is performed in a continuous method, and a linear velocity of the internal olefin sulfonate composition is preferably 0.001 mm/s or more, more preferably 0.005 mm/s or more, still more preferably 0.01 mm/s or more, and yet still more preferably 0.1 mm/s or more, and is preferably 1,000 mm/s or less, more preferably 800 mm/s or less, still more preferably 500 mm/s or less, yet still more preferably 300 mm/s or less, yet still more preferably 100 mm/s or less, yet still more preferably 50 mm/s or less, yet still more preferably 10 mm/s or less, yet still more preferably 5 mm/s or less, and yet still more preferably 1 mm/s or less.

<61> The method for producing a decolorized internal olefin sulfonate composition according to any one of <1> to <60>, wherein a fading rate of the decolorized internal olefin sulfonate composition obtained in the decolorizing process (A) with respect to the internal olefin sulfonate composition before decolorizing is preferably 50% or more, more preferably 60% or more, still more preferably 63% or more, and yet still more preferably 65% or more.

<62> The method for producing a decolorized internal olefin sulfonate composition according to any one of <1> to <61>, wherein a peroxide value of the decolorized internal olefin sulfonate composition obtained in the decolorizing process (A) is preferably 37.0 meq or less, more preferably 30.0 meq or less, still more preferably 20.0 meq or less, yet still more preferably 10 meq or less, yet still more preferably 7 meq or less, and yet still more preferably 5 meq or less with respect to 1 kg of the internal olefin sulfonate in the internal olefin sulfonate composition.

<63> The method for producing a decolorized internal olefin sulfonate composition according to any one of <1> to <62>, further containing a pH adjusting step.

<64> The method for producing a decolorized internal olefin sulfonate composition according to <63>, wherein a pH of the internal olefin sulfonate composition after pH adjustment is preferably 8.5 or more, and more preferably 9.0 or more, and is preferably 11.0 or less, and more preferably 10.5 or less.

<65> The method for producing a decolorized internal olefin sulfonate composition according to <63> or <64>, wherein an acid used for pH adjustment is preferably at least one selected from the group consisting of citric acid and phosphoric acid, and more preferably citric acid.

<66> The method for producing a decolorized internal olefin sulfonate composition according to any one of <1> to <65>,

   wherein the internal olefin sulfonate composition is obtained by a method for producing an internal olefin sulfonate including a sulfonation process of sulfonating a raw material olefin, a neutralization process of neutralizing a sulfonated product obtained, and a hydrolysis process of hydrolyzing a neutralization product obtained, and the number of carbon atoms in the internal olefin is preferably 10 or more, more preferably 12 or more, and still more preferably 14 or more, and is preferably 22 or less, and more preferably 18 or less.

<67> The method for producing a decolorized internal olefin sulfonate composition according to <66>, wherein the internal olefin is preferably at least one selected from the group consisting of an internal olefin having 16 carbon atoms and an internal olefin having 18 carbon atoms.

<68> The method for producing a decolorized internal olefin sulfonate composition according to <66> or <67>, wherein a treatment temperature in the sulfonation process is preferably 0°C or higher, and is preferably 50°C or lower.

<69> The method for producing a decolorized internal olefin sulfonate composition according to any one of <66> to <68>, wherein a concentration of the sulfur trioxide gas in the treatment of the sulfonation process is preferably 0.5 vol% or more, and more preferably 1.0 vol% or more, and is preferably 10 vol% or less, and more preferably 6.0 vol% or less.

<70> The method for producing a decolorized internal olefin sulfonate composition according to any one of <66> to <69>, wherein an amount of sulfur trioxide used in the treatment of the sulfonation process is preferably 0.8 mol or more, more preferably 0.9 mol or more, and still more preferably 0.95 mol or more, and is preferably 1.2 mol or less, more preferably 1.10 mol or less, and still more preferably 1.05 mol or less, based on 1 mol of the internal olefin.

<71> The method for producing a decolorized internal olefin sulfonate composition according to any one of <66> to <70>, wherein a sulfonation reactor used in the sulfonation process is preferably a reactor including an external jacket and more preferably a falling thin-film type sulfonation reactor in which the internal olefin flows down in a thin-film form and reacts with the sulfur trioxide gas.

<72> The method for producing a decolorized internal olefin sulfonate composition according to <71>, wherein the sulfonation reactor has an external jacket and is cooled by passing cooling water through the external jacket.

<73> The method for producing a decolorized internal olefin sulfonate composition according to <72>, wherein a temperature of the cooling water, which is passed into the external jacket of the sulfonation reactor is preferably 0 °C or higher, and is preferably 30°C or lower, and more preferably 20°C or lower.

<74> The method for producing a decolorized internal olefin sulfonate composition according to any one of <66> to <73>, wherein a reaction rate of the internal olefin in the sulfonation reaction is preferably 95% or more, more preferably 96% or more, and still more preferably 97% or more, and is preferably 99.8% or less.

<75> The method for producing a decolorized internal olefin sulfonate composition according to any one of <66> to <74>, wherein an alkali compound used for neutralization is preferably an inorganic alkali compound, more preferably alkali metal hydroxides, and more preferably at least one selected from the group consisting of sodium hydroxide and potassium hydroxide.

<76> The method for producing a decolorized internal olefin sulfonate composition according to any one of <66> to <75>, wherein the alkali compound used for neutralization is an aqueous alkali solution, and a concentration of the aqueous alkali solution or a concentration of a mixed solution when the aqueous alkali solution and water are mixed is preferably 1% by mass or more, more preferably 4.5% by mass or more, still more preferably 7% by mass or more, yet still more preferably 10% by mass or more, and yet still more preferably 12% by mass or more, and is preferably 50% by mass or less, more preferably 32% by mass or less, still more preferably 23% by mass or less, yet still more preferably 20% by mass or less, and yet still more preferably 18% by mass or less.

<77> The method for producing a decolorized internal olefin sulfonate composition according to any one of <66> to <76>, wherein the concentration of the alkali compound in the internal olefin sulfonate composition after the hydrolysis process is controlled by adjusting the amount of the alkali compound used in the neutralization process and/or the hydrolysis process.

<78> The method for producing a decolorized internal olefin sulfonate composition according to any one of <66> to <77>, wherein an amount of the alkali compound used in the neutralization process is preferably 1 time by mole or more, more preferably 1.02 times by mole or more, still more preferably 1.03 times by mole or more, yet still more preferably 1.034 times by mole or more, yet still more preferably 1.035 times by mole or more, yet still more preferably 1.040 times by mole or more, with respect to a sulfonic acid group, and is preferably 1.13 times by mole or less, more preferably 1.12 times by mole or less, still more preferably 1.110 times by mole or less, yet still more preferably 1.105 times by mole or less, yet still more preferably 1.10 times by mole or less, yet still more preferably 1.09 times by mole or less, yet still more preferably 1.08 times by mole or less, yet still more preferably 1.07 times by mole or less, and yet still more preferably 1.06 times by mole or less.

<79> The method for producing a decolorized internal olefin sulfonate composition according to any one of <66> to <78>, wherein in the neutralization process, a temperature when the sulfonate is mixed with the aqueous alkali solution and a temperature during the neutralization reaction are preferably 40°C or lower, more preferably 35°C or lower, still more preferably 30°C or lower, and yet still more preferably 25°C or lower, and is preferably 0°C or higher, more preferably 10°C or higher, still more preferably 15°C or higher, and yet still more preferably 20°C or higher.

<80> The method for producing a decolorized internal olefin sulfonate composition according to any one of <66> to <79>, wherein a mixer used in the neutralization process is an agitating impeller type mixer.

<81> The method for producing a decolorized internal olefin sulfonate composition according to any one of <66> to <80>, wherein an agitating speed of the agitator of the agitating impeller type mixer is preferably 5 m/s or more, more preferably 10 m/s or more, and still more preferably 20 m/s or more, and is preferably 50 m/s or less, more preferably 30 m/s or less, still more preferably 27.5 m/s or less, and yet still more preferably 25 m/s or less.

<82> The method for producing a decolorized internal olefin sulfonate composition according to any one of <66> to <81>, wherein the neutralization process is performed in a continuous method in which a sulfonated product and an aqueous alkali solution are added while a reaction liquid is circulated using a loop type reaction device, and the reaction liquid is simultaneously extracted.

<83> The method for producing a decolorized internal olefin sulfonate composition according to any one of <66> to <82>, wherein in the neutralization process, a mixing time when the sulfonated product and the aqueous alkali solution are mixed is preferably 10 minutes or more, more preferably 20 minutes or more, and still more preferably 30 minutes or more, and is preferably 120 minutes or less, more preferably 90 minutes or less, still more preferably 60 minutes or less, and yet still more preferably 50 minutes or less.

<84> The method for producing a decolorized internal olefin sulfonate composition according to any one of <66> to <83>, wherein a concentration of the internal olefin sulfonate in the neutralization product is preferably 40% by mass or more, more preferably 45% by mass or more, and still more preferably 48% by mass or more, and is preferably 65% by mass or less, and more preferably 60% by mass or less.

<85> The method for producing a decolorized internal olefin sulfonate composition according to any one of <66> to <84>, wherein a temperature during the hydrolysis in the hydrolysis process is preferably 120°C or higher, more preferably 140°C or higher, and still more preferably 160°C or higher, and is preferably 220°C or lower, and more preferably 200°C or lower.

<86> The method for producing a decolorized internal olefin sulfonate composition according to any one of <66> to <85>, wherein a treatment time of the hydrolysis process is preferably 30 minutes or more, more preferably 45 minutes or more, and still more preferably 1 hour or more, and is preferably 4 hours or less, and more preferably 3 hours or less.

<87> The method for producing a decolorized internal olefin sulfonate composition according to any one of <66> to <86>, wherein an average double bond position of the raw material olefin of the internal olefin sulfonate is preferably 3 or more, more preferably 3.5 or more, and still more preferably 3.8 or more, and is preferably 5 or less, more preferably 4.5 or less, still more preferably 4.4 or less, and yet still more preferably 4.3 or less.

<88> The method for producing a decolorized internal olefin sulfonate composition according to any one of <1> to <87>, wherein the internal olefin sulfonate preferably contains one or more internal olefin sulfonates selected from the group consisting of an internal olefin sulfonate having 16 carbon atoms and an internal olefin sulfonate having 18 carbon atoms.

<89> The method for producing a decolorized internal olefin sulfonate composition according to any one of <66> to <88>, wherein a concentration of the alkali compound in the internal olefin sulfonate composition obtained in the hydrolysis process and/or the internal olefin sulfonate composition before the decolorizing process (A) is preferably 0.010 mmol or more, more preferably 0.015 mmol or more, still more preferably 0.020 mmol or more, yet still more preferably 0.025 mmol or more, and yet still more preferably 0.029 mmol or more, and is preferably 0.27 mmol or less, more preferably 0.240 mmol or less, and still more preferably 0.210 mmol or less, and is preferably 0.27 mmol or less, more preferably 0.240 mmol or less, still more preferably 0.220 mmol or less, yet still more preferably 0.210 mmol or less, yet still more preferably 0.20 mmol or less, yet still more preferably 0.18 mmol or less, yet still more preferably 0.16 mmol or less, yet still more preferably 0.10 mmol or less, and yet still more preferably 0.08 mmol or less, with respect to 1 g of the internal olefin sulfonate in the internal olefin sulfonate composition.

<90> A method for producing a decolorized internal olefin sulfonate composition, the method including a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

wherein the internal olefin sulfonate after the hydrolysis process contains at least one selected from the group consisting of an internal olefin sulfonate having 16 carbon atoms and an internal olefin sulfonate having 18 carbon atoms, and an average double bond position of a raw material olefin of the internal olefin sulfonate is 3.8 or more and 4.4 or less,

the alkali compound to be used for neutralization is at least one selected from sodium hydroxide and potassium hydroxide, the agitating speed of an agitating impeller type mixer in the neutralization process is 20 m/s or more and 200 m/s or less, the concentration of the internal olefin sulfonate in a neutralization product is 45% by mass or more and 65% by mass or less, a temperature at the time of the neutralization reaction is 0°C or higher and 35°C or lower, neutralization is performed for a residence time in a neutralization device of 30 minutes or more and 120 minutes or less, and the concentration of the alkali compound of the internal olefin sulfonate composition is controlled by adjusting the amount of the alkali compound used in the neutralization process,

the concentration of the alkali compound in the internal olefin sulfonate composition after the hydrolysis process is controlled by a method for adjusting the amount of the alkali compound used in the neutralization process,

a temperature during hydrolysis in the hydrolysis process is 160°C or higher and 220°C or lower, and a treatment time in the hydrolysis process is 1 hour or more and 4 hours or less,

the adjusting step (a1) is performed in a continuous method, a circulation ratio is greater than 0 and 20 or less, a reaction tank having an agitating impeller, or an agitating impeller type mixer is used in the adjusting step (a1), d/D is 0.01 or more and less than 1, h/H is 0.01 or more and less than 1, and the concentration of the alkali compound in the system in the adjusting step (a1) is adjusted to 0.005 mmol or more and 0.26 mmol or less with respect to 1 g of the internal olefin sulfonate after the mixing step (a2),

the mixing step (a2) is performed in a continuous method, the pH of the liquid in the system in the mixing step (a2) is 9.3 or more and 11.4 or less, the reaction tank having the agitating impeller or the agitating impeller type mixer is used in the mixing step (a2), the d/D is 0.01 or more and less than 1, the h/H is 0.01 or more and less than 1, a peripheral speed of the agitating impeller is 0.01 m/s or more and 50 m/s or less, and the pressure in the system is 0 MPaG or more and 10 MPaG or less, and

the aging step (b) is performed in a continuous method, a temperature range is 40°C or higher and 100°C or lower, a residence time is 10 minutes or more and 500 minutes or less, the aging step (b) is performed using a vertical cylindrical reactor, $H_1/D_1$ is 0.1 or more and 300 or less, a linear velocity of the internal olefin sulfonate composition is 0.01 mm/s or more and 1,000 mm/s or less, and a pressure in the aging step (b) is 0 MPaG or more and 10 MPaG or less.

<91> A method for producing a decolorized internal olefin sulfonate composition, the method including a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

wherein the internal olefin sulfonate after the hydrolysis process contains at least one or more selected from the group consisting of an internal olefin sulfonate having 16 carbon atoms and an internal olefin sulfonate having 18 carbon atoms, and an average double bond position of a raw material olefin of the internal olefin sulfonate is 3.8 or more and 4.4 or less,

the alkali compound to be used for neutralization is at least one selected from sodium hydroxide and potassium hydroxide, the agitating speed of an agitating impeller type mixer in the neutralization process is 20 m/s or more and 200 m/s or less, the concentration of the internal olefin sulfonate in a neutralization product is 45% by mass or more and 65% by mass or less, a temperature at the time of the neutralization reaction is 0°C or higher and 35°C or lower, neutralization is performed for a residence time in a neutralization device of 30 minutes or more and 120 minutes or less, and the concentration of the alkali compound of the internal olefin sulfonate composition is controlled by adjusting the amount of the alkali compound used in the neutralization process,

the concentration of the alkali compound in the internal olefin sulfonate composition after the hydrolysis process is controlled by a method for adjusting the amount of the alkali compound used in the neutralization process,

a temperature during hydrolysis in the hydrolysis process is 160°C or higher and 220°C or lower, and a treatment time in the hydrolysis process is 1 hour or more and 4 hours or less,
the adjusting step (a1) is performed in a continuous method, a circulation ratio is greater than 0 and 20 or less, a reaction tank having an agitating impeller, or an agitating impeller type mixer is used in the adjusting step (a1), d/D is 0.01 or more and less than 1, h/H is 0.01 or more and less than 1, and the concentration of the alkali compound in the system in the adjusting step (a1) is adjusted to 0.005 mmol or more and 0.26 mmol or less with respect to 1 g of the internal olefin sulfonate after the mixing step (a2),
the mixing step (a2) is performed in a continuous method, the pH of the liquid in the system in the mixing step (a2) is 9.3 or more and 11.4 or less, the reaction tank having the agitating impeller or the agitating impeller type mixer is used in the mixing step (a2), the d/D is 0.01 or more and less than 1, the h/H is 0.01 or more and less than 1, the shear rate is 5/s or more and 100,000 /s or less, and the pressure in the system is 0 MPaG or more and 10 MPaG or less, and
the aging step (b) is performed in a continuous method, a temperature range is 40°C or higher and 100°C or lower, a residence time is 10 minutes or more and 500 minutes or less, the aging step (b) is performed using a vertical cylindrical reactor, $H_1/D_1$ is 0.1 or more and 300 or less, a linear velocity of the internal olefin sulfonate composition is 0.01 mm/s or more and 1,000 mm/s or less, and a pressure in the aging step (b) is 0 MPaG or more and 10 MPaG or less.

<92> A method for producing a decolorized internal olefin sulfonate composition, the method including a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

wherein the internal olefin sulfonate after the hydrolysis process contains at least one or more selected from the group consisting of an internal olefin sulfonate having 16 carbon atoms and an internal olefin sulfonate having 18 carbon atoms, and an average double bond position of a raw material olefin of the internal olefin sulfonate is 3.8 or more and 4.4 or less,
the alkali compound to be used for neutralization is at least one selected from sodium hydroxide and potassium hydroxide, the agitating speed of an agitating impeller type mixer in the neutralization process is 20 m/s or more and 200 m/s or less, the concentration of the internal olefin sulfonate in a neutralization product is 45% by mass or more and 65% by mass or less, a temperature at the time of the neutralization reaction is 0°C or higher and 35°C or lower, neutralization is performed for a residence time in a neutralization device of 30 minutes or more and 120 minutes or less, and the concentration of the alkali compound of the internal olefin sulfonate composition is controlled by adjusting the amount of the alkali compound used in the neutralization process,
the concentration of the alkali compound in the internal olefin sulfonate composition after the hydrolysis process is controlled by a method for adjusting the amount of the alkali compound used in the neutralization process,
a temperature during hydrolysis in the hydrolysis process is 160°C or higher and 220°C or lower, and a treatment time in the hydrolysis process is 1 hour or more and 4 hours or less,
the adjusting step (a1) is performed in a continuous method, a circulation ratio is greater than 0 and 20 or less, a reaction tank having an agitating impeller, or an agitating impeller type mixer is used in the adjusting step (a1), d/D is 0.01 or more and less than 1, h/H is 0.01 or more and less than 1, and the concentration of the alkali compound in the system in the adjusting step (a1) is adjusted to 0.005 mmol or more and 0.26 mmol or less with respect to 1 g of the internal olefin sulfonate after the mixing step (a2),
the mixing step (a2) is performed in a continuous method, the pH of the liquid in the system in the mixing step (a2) is 9.3 or more and 11.4 or less, the reaction tank having the agitating impeller or the agitating impeller type mixer is used in the mixing step (a2), the d/D is 0.01 or more and less than 1, the h/H is 0.01 or more and less than 1, the stirring power per unit liquid amount (Pv) is 0.1 kW/m$^3$ or more and 100 kW/m$^3$ or less, and the pressure in the system is 0 MPaG or more and 10 MPaG or less, and
the aging step (b) is performed in a continuous method, a temperature range is 40°C or higher and 100°C or lower, a residence time is 10 minutes or more and 500 minutes or less, the aging step (b) is performed using a vertical cylindrical reactor, $H_1/D_1$ is 0.1 or more and 300 or less, a linear velocity of the internal olefin sulfonate composition is 0.01 mm/s or more and 1,000 mm/s or less, and a pressure in the aging step (b) is 0 MPaG or more and 10 MPaG or less.

<93> A method for producing a decolorized internal olefin sulfonate composition, the method including a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the number of carbon atoms of the internal olefin sulfonate after the hydrolysis process is 10 or more and 22 or less, the average double bond position of the internal olefin sulfonate is 3 or more and 5 or less, the alkali compound used for neutralization is an inorganic alkali compound, the agitating speed of an agitating impeller type mixer in the neutralization process is 5 m/s or more and 200 m/s or less, the concentration of the internal olefin sulfonate in a neutralization product is 40% by mass or more and 65% by mass or less, a temperature during the neutralization reaction is 0°C or higher and 40°C or lower, neutralization is performed for a residence time in a neutralization device of 10 minutes or more and 120 minutes or less, and the concentration of the alkali compound in the internal olefin sulfonate composition after the hydrolysis process is controlled by an adjusting method selected from a method for adjusting the amount of the alkali compound used in the neutralization process and a method for adjusting the amount of the alkali compound in the hydrolysis process,
a temperature during hydrolysis in the hydrolysis process is 120°C or higher and 220°C or lower, and a treatment time in the hydrolysis process is 30 minutes or more and 4 hours or less,
the adjusting step (a1) is performed in a batch, semi-batch, or continuous method, a circulation ratio is greater than 0 and 20 or less, a reaction tank having an agitating impeller, or an agitating impeller type mixer is used in the adjusting step (a1), d/D is 0.01 or more and less than 1, h/H is 0.01 or more and less than 1, and the concentration of the alkali compound in the system in the adjusting step (a1) is adjusted to 0.005 mmol or more and 0.26 mmol or less with respect to 1 g of the internal olefin sulfonate after the mixing step (a2),
the mixing step (a2) is performed in a continuous method, the pH of the liquid in the system in the mixing step (a2) is 9.3 or more and 11.4 or less, the reaction tank having the agitating impeller or the agitating impeller type mixer is used in the mixing step (a2), the d/D is 0.01 or more and less than 1, the h/H is 0.01 or more and less than 1, a peripheral speed of the agitating impeller is 0.01 m/s or more and 50 m/s or less, and the pressure in the system is 0 MPaG or more and 10 MPaG or less, and
the aging step (b) is performed in a continuous method, a temperature range is 40°C or higher and 120°C or lower, a residence time is 10 minutes or more and 500 minutes or less, the aging step (b) is performed using a vertical cylindrical reactor, $H_1/D_1$ is 0.1 or more and 300 or less, a linear velocity of the internal olefin sulfonate composition is 0.001 mm/s or more and 1,000 mm/s or less, and a pressure in the aging step (b) is 0 MPaG or more and 10 MPaG or less.

<94> A method for producing a decolorized internal olefin sulfonate composition, the method including a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the number of carbon atoms of the internal olefin sulfonate after the hydrolysis process is 10 or more and 22 or less, the average double bond position of the internal olefin sulfonate is 3 or more and 5 or less, the alkali compound used for neutralization is an inorganic alkali compound, the agitating speed of an agitating impeller type mixer in the neutralization process is 5 m/s or more and 200 m/s or less, the concentration of the internal olefin sulfonate in a neutralization product is 40% by mass or more and 65% by mass or less, a temperature during the neutralization reaction is 0°C or higher and 40°C or lower, neutralization is performed for a residence time in a neutralization device of 10 minutes or more and 120 minutes or less, and the concentration of the alkali compound in the internal olefin sulfonate composition after the hydrolysis process is controlled by an adjusting method selected from a method for adjusting the amount of the alkali compound used in the neutralization process and a method for adjusting the amount of the alkali compound in the hydrolysis process,
a temperature during hydrolysis in the hydrolysis process is 120°C or higher and 220°C or lower, and a treatment time in the hydrolysis process is 30 minutes or more and 4 hours or less,
the adjusting step (a1) is performed in a batch, semi-batch, or continuous method, a circulation ratio is greater than 0 and 20 or less, a reaction tank having an agitating impeller, or an agitating impeller type mixer is used in the adjusting step (a1), d/D is 0.01 or more and less than 1, h/H is 0.01 or more and less than 1, and the concentration of the alkali compound in the system in the adjusting step (a1) is adjusted to 0.005 mmol or more and 0.26 mmol or less with respect to 1 g of the internal olefin sulfonate after the mixing step (a2),

the mixing step (a2) is performed in a continuous method, the pH of the liquid in the system in the mixing step (a2) is 9.3 or more and 11.4 or less, the reaction tank having the agitating impeller or the agitating impeller type mixer is used in the mixing step (a2), the d/D is 0.01 or more and less than 1, the h/H is 0.01 or more and less than 1, the shear rate is 5/s or more and *100,000* /s or less, and the pressure in the system is 0 MPaG or more and 10 MPaG or less, and

the aging step (b) is performed in a continuous method, a temperature range is 40°C or higher and 120°C or lower, a residence time is 10 minutes or more and 500 minutes or less, the aging step (b) is performed using a vertical cylindrical reactor, $H_1/D_1$ is 0.1 or more and 300 or less, a linear velocity of the internal olefin sulfonate composition is 0.001 mm/s or more and 1,000 mm/s or less, and a pressure in the aging step (b) is 0 MPaG or more and 10 MPaG or less.

<95> A method for producing a decolorized internal olefin sulfonate composition, the method including a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the number of carbon atoms of the internal olefin sulfonate after the hydrolysis process is 10 or more and 22 or less, the average double bond position of the internal olefin sulfonate is 3 or more and 5 or less, the alkali compound used for neutralization is an inorganic alkali compound, the agitating speed of an agitating impeller type mixer in the neutralization process is 5 m/s or more and 200 m/s or less, the concentration of the internal olefin sulfonate in a neutralization product is 40% by mass or more and 65% by mass or less, a temperature during the neutralization reaction is 0°C or higher and 40°C or lower, neutralization is performed for a residence time in a neutralization device of 10 minutes or more and 120 minutes or less, and the concentration of the alkali compound in the internal olefin sulfonate composition after the hydrolysis process is controlled by an adjusting method selected from a method for adjusting the amount of the alkali compound used in the neutralization process and a method for adjusting the amount of the alkali compound in the hydrolysis process,

a temperature during hydrolysis in the hydrolysis process is 120°C or higher and 220°C or lower, and a treatment time in the hydrolysis process is 30 minutes or more and 4 hours or less,

the adjusting step (a1) is performed in a batch, semi-batch, or continuous method, a circulation ratio is greater than 0 and 20 or less, a reaction tank having an agitating impeller, or an agitating impeller type mixer is used in the adjusting step (a1), d/D is 0.01 or more and less than 1, h/H is 0.01 or more and less than 1, and the concentration of the alkali compound in the system in the adjusting step (a1) is adjusted to 0.005 mmol or more and 0.26 mmol or less with respect to 1 g of the internal olefin sulfonate after the mixing step (a2),

the mixing step (a2) is performed in a continuous method, the pH of the liquid in the system in the mixing step (a2) is 9.3 or more and 11.4 or less, the reaction tank having the agitating impeller or the agitating impeller type mixer is used in the mixing step (a2), the d/D is 0.01 or more and less than 1, the h/H is 0.01 or more and less than 1, the stirring power per unit liquid amount (Pv) is 0.1 kW/m$^3$ or more and 100 kW/m$^3$ or less, and the pressure in the system is 0 MPaG or more and 10 MPaG or less, and

the aging step (b) is performed in a continuous method, a temperature range is 40°C or higher and 120°C or lower, a residence time is 10 minutes or more and 500 minutes or less, the aging step (b) is performed using a vertical cylindrical reactor, $H_1/D_1$ is 0.1 or more and 300 or less, a linear velocity of the internal olefin sulfonate composition is 0.001 mm/s or more and 1,000 mm/s or less, and a pressure in the aging step (b) is 0 MPaG or more and 10 MPaG or less.

<96> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the stirring power per unit liquid amount (Pv) of the mixing step (a2) is 0.1 kW/m$^3$ or more and 100 kW/m$^3$ or less.

<97> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
the stirring power per unit liquid amount (Pv) of the mixing step (a2) is 0.3 kW/m$^3$ or more and 30 kW/m$^3$ or less.

<98> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
the stirring power per unit liquid amount (Pv) of the mixing step (a2) is 0.2 kW/m$^3$ or more and 50 kW/m$^3$ or less.

<99> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
the shear rate of the mixing step (a2) is 5/s or more and 100,000/s or less.

<100> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
the shear rate of the mixing step (a2) is 1,500/s or more and 20,000/s or less.

<101> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
the shear rate of the mixing step (a2) is 500/s or more and 50,000/s or less.

<102> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a peripheral speed of the agitating impeller is 0.01 m/s or more and 50 m/s or less, when the reaction tank having the agitating impeller or the agitating impeller type mixer is used in the mixing step (a2).

<103> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a peripheral speed of the agitating impeller is 9.0 m/s or more and 15 m/s or less, when the reaction tank having the agitating impeller or the agitating impeller type mixer is used in the mixing step (a2).

<104> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a peripheral speed of the agitating impeller is 0.9 m/s or more and 26 m/s or less, when the reaction tank having the agitating impeller or the agitating impeller type mixer is used in the mixing step (a2).

<105> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), a peripheral speed of the agitating impeller is 0.01 m/s or more and 50 m/s or less when the reaction tank having the agitating impeller or the agitating impeller type mixer is used in the mixing step (a2), and

the concentration of the alkali compound in the adjusting step (a1) is 0.005 mmol or more and 0.23 mmol or less with respect to 1 g of the internal olefin sulfonate after the mixing step (a2).

<106> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), a peripheral speed of the agitating impeller is 0.01 m/s or more and 50 m/s or less, when the reaction tank having the agitating impeller or the agitating impeller type mixer is used in the mixing step (a2), and

the concentration of the alkali compound in the adjusting step (a1) is 0.030 mmol or more and 0.06 mmol or less with respect to 1 g of the internal olefin sulfonate after the mixing step (a2).

<107> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less, when the reaction tank having the agitating impeller or the agitating impeller type mixer is used in the mixing step (a2), and
the concentration of the alkali compound in the adjusting step (a1) is 0.020 mmol or more and 0.20 mmol or less with respect to 1 g of the internal olefin sulfonate after the mixing step (a2).

<108> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and the pH of the liquid in the system in the mixing step (a2) is 9.3 or more and 11.4 or less.

<109> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less, when the reaction tank having the agitating impeller or the agitating impeller type mixer is used in the mixing step (a2), and the pH of the liquid in the system in the mixing step (a2) is 10.0 or more and 10.5 or less.

<110> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less, when the reaction tank having the agitating impeller or the agitating impeller type mixer is used in the mixing step (a2), and the pH of the liquid in the system in the mixing step (a2) is 9.8 or more and 11.0 or less.

<111> The method for producing a decolorized internal olefin sulfonate composition according to any one of <1> to <89>,

EP 4 755 872 A1

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and
the concentration of the alkali compound in the adjusting step (a1) is 0.005 mmol or more and 0.23 mmol or less with respect to 1 g of the internal olefin sulfonate after the mixing step (a2), and the adjusting step (a1) is performed in a batch method.

<112> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and
the concentration of the alkali compound in the adjusting step (a1) is 0.005 mmol or more and 0.23 mmol or less with respect to 1 g of the internal olefin sulfonate after the mixing step (a2), and the adjusting step (a1) is performed in a semi-batch method.

<113> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and
the concentration of the alkali compound in the adjusting step (a1) is 0.005 mmol or more and 0.23 mmol or less with respect to 1 g of the internal olefin sulfonate after the mixing step (a2), and the adjusting step (a1) is performed in a continuous method.

<114> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method,
the concentration of the alkali compound in the adjusting step (a1) is 0.005 mmol or more and 0.23 mmol or less with respect to 1 g of the internal olefin sulfonate after the mixing step (a2), and
the adjusting step (a1) is performed in a batch method, a semi-batch method, or a continuous method.

<115> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), and the mixing step (a2) is performed in a semi-batch method, and

the concentration of the alkali compound in the adjusting step (a1) is 0.005 mmol or more and 0.23 mmol or less with respect to 1 g of the internal olefin sulfonate after the mixing step (a2), and the adjusting step (a1) is performed in a batch method, a semi-batch method, or a continuous method.

<116> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), and the mixing step (a2) is performed in a continuous method, and

the concentration of the alkali compound in the adjusting step (a1) is 0.005 mmol or more and 0.23 mmol or less with respect to 1 g of the internal olefin sulfonate after the mixing step (a2), and the adjusting step (a1) is performed in a batch method, a semi-batch method, or a continuous method.

<117> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method, and

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less.

<118> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method, and
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is 4 times or more and 8 times or less.

<119> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method, and
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is 2 times or more and 10 times or less.

<120> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and
the aging step (b) is performed in a continuous method, and a linear velocity of the internal olefin sulfonate composition is 0.001 mm/s or more and 1,000 mm/s or less.

<121> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method,
the concentration of the alkali compound in the adjusting step (a1) is 0.005 mmol or more and 0.23 mmol or less with respect to 1 g of the internal olefin sulfonate after the mixing step (a2), and the adjusting step (a1) is performed in a continuous method, and a circulation ratio is greater than 0 times and 20 times or less, and
the aging step (b) is performed in a continuous method, and a linear velocity of the internal olefin sulfonate composition is 0.1 mm/s or more and 1 mm/s or less.

# EP 4 755 872 A1

<122> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 100 m/s or less when the reaction tank having the agitating impeller or the agitating impeller type mixer is used in the mixing step (a2), and the mixing step (a2) is performed in a batch method, a semi-batch method, or a continuous method,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and
the aging step (b) is performed in a continuous method, and a linear velocity of the internal olefin sulfonate composition is 0.005 mm/s or more and 800 mm/s or less.

<123> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and
the aging step (b) is performed in a continuous method, a linear velocity of the internal olefin sulfonate composition is 0.001 mm/s or more and 1,000 mm/s or less, the aging step (b) is performed using the vertical cylindrical reactor, and a ratio ($H_1/D_1$) of the height $H_1$ of the vertical cylindrical reactor to the inner diameter $D_1$ of the reactor is 0.1 or more and 300 or less.

<124> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the aging step (b) is performed in a continuous method, and a linear velocity of the internal olefin sulfonate composition is 0.001 mm/s or more and 1,000 mm/s or less, and
the aging step (b) is performed using the vertical cylindrical reactor, and a ratio ($H_1/D_1$) of the height $H_1$ of the vertical cylindrical reactor to the inner diameter $D_1$ of the reactor is 1.5 or more and 5 or less.

31

<125> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), and the mixing step (a2) is performed in a batch method, a semi-batch method, or a continuous method,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the aging step (b) is performed in a continuous method, and a linear velocity of the internal olefin sulfonate composition is 0.001 mm/s or more and 1,000 mm/s or less, and

the aging step (b) is performed using the vertical cylindrical reactor, and a ratio ($H_1/D_1$) of the height $H_1$ of the vertical cylindrical reactor to the inner diameter $D_1$ of the reactor is 1 or more and 50 or less.

<126> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less,

the aging step (b) is performed in a continuous method, and a linear velocity of the internal olefin sulfonate composition is 0.001 mm/s or more and 1,000 mm/s or less, and

the aging step (b) is performed using the vertical cylindrical reactor, and a ratio ($H_1/D_1$) of the height $H_1$ of the vertical cylindrical reactor to the inner diameter $D_1$ of the reactor is 0.1 or more and 300 or less.

<127> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is hydrogen peroxide,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, and a ratio ($H_1/D_1$) of the height $H_1$ of the vertical cylindrical reactor to the inner diameter $D_1$ of the reactor is 0.1 or more and 300 or less.

<128> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, and a pressure in the aging step (b) is 0 MPaG or more and 10 MPaG or less.

<129> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, and a pressure in the aging step (b) is 0.2 MPaG or more and 0.5 MPaG or less.

<130> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, and a pressure in the aging step (b) is 0.05 MPaG or more and 1 MPaG or less.

<131> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the aging step (b) is performed in a continuous method, and a linear velocity of the internal olefin sulfonate composition is 0.001 mm/s or more and 1,000 mm/s or less, and
the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, and a residence time in the aging step (b) is 10 minutes or more and 500 minutes or less.

<132> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, and a residence time in the aging step (b) is 60 minutes or more and 200 minutes or less.

<133> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, and a residence time in the aging step (b) is 30 minutes or more and 400 minutes or less.

<134> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<135> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 60°C or higher and 100°C or lower.

<136> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, and a circulation ratio is greater than 0 times and 20 times or less,

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 50°C or higher and 110°C or lower.

<137> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, and the pressure in the system in the adjusting step (a1) is 0 MPaG or more and 10 MPaG or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<138> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, and the pressure in the system in the adjusting step (a1) is 0.2 MPaG or more and 0.5 MPaG or less, and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<139> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, and the decolorizing agent is at least one selected from the group consisting of hydrogen peroxide, hypochlorous acid, nitric acid, potassium nitrate, permanganate, peroxide, chlorite, chlorate, perchlorate, and persulfate,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, and the pressure in the system in the adjusting step (a1) is 0.05 MPaG or more and 1 MPaG or less, and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<140> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0 MPaG or more and 10 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, and the pressure in the system in the adjusting step (a1) is 0 MPaG or more and 10 MPaG or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<141> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0.2 MPaG or more and 0.5 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, and the pressure in the system in the adjusting step (a1) is 0 MPaG or more and 10 MPaG or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<142> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0.05 MPaG or more and 5 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, and the pressure in the system in the adjusting step (a1) is 0 MPaG or more and 10 MPaG or less, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<143> The method for producing a decolorized internal olefin sulfonate composition according to any one of <1> to <89>,

wherein the method includes a sulfonation step of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0 MPaG or more and 10 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), a ratio (d/D) of an impeller diameter d to a tank inner diameter D is 0.01 or more and less than 1, and a ratio (h/H) of an agitating impeller height h to a tank height H of the reaction tank is 0.01 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<144> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0 MPaG or more and 10 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 9.0 m/s or more and 15 m/s or less when the reaction tank having the agitating impeller or the agitating impeller type mixer is used in the adjusting step (a1), a ratio (d/D) of an impeller diameter d to a tank inner diameter D is 0.55 or more and less than 1, and a ratio (h/H) of an agitating impeller height h to a tank height H of the reaction tank is 0.5 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<145> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0 MPaG or more and 10 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.3 m/s or more and 26 m/s or less when the reaction tank having the agitating impeller or the agitating impeller type mixer is used in the adjusting step (a1), and a ratio (d/D) of an impeller diameter d to a tank inner diameter D is 0.1 or more and less than 1, and a ratio (h/H) of an agitating impeller height h to a tank height H of the reaction tank is 0.1 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<146> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0 MPaG or more and 10 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), and a ratio (d/D) of an impeller diameter d to a tank inner diameter D is 0.5 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio $(H_1/D_1)$ of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<147> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

a peripheral speed of the agitating impeller is 0.01 m/s or more and 50 m/s or less when the reaction tank having the agitating impeller or the agitating impeller type mixer is used in the mixing step (a2), the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, and the pressure in the system in the mixing step (a2) is 0 MPaG or more and 10 MPaG or less,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), and a ratio (h/H) of an agitating impeller height h to a tank height H of the reaction tank is 0.3 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio $(H_1/D_1)$ of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<148> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, the pressure in the system in the mixing step (a2) is 0 MPaG or more and 10 MPaG or less, a peripheral speed of the agitating impeller is 0.01 m/s or more and 50 m/s or less when the reaction tank having the agitating impeller or the agitating impeller type mixer is used in the mixing step (a2), a ratio (d/D) of an impeller diameter d to a tank inner diameter D is 0.01 or more and less than 1, and a ratio (h/H) of an agitating impeller height h to a tank height H of the reaction tank is 0.01 or more and less than 1,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), a ratio (d/D) of an impeller diameter d to a tank inner diameter D is 0.01 or more and less than 1, and a ratio (h/H) of an agitating impeller height h to a tank height H of the reaction tank is 0.01 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<149> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, the pressure in the system in the mixing step (a2) is 0 MPaG or more and 10 MPaG or less, a peripheral speed of the agitating impeller is 9.0 m/s or more and 15 m/s or less when the reaction tank having the agitating impeller or the agitating impeller type mixer is used in the mixing step (a2), a ratio (d/D) of an impeller diameter d to a tank inner diameter D is 0.55 or more and less than 1, and a ratio (h/H) of an agitating impeller height h to a tank height H of the reaction tank is 0.55 or more and less than 1,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), a ratio (d/D) of an impeller diameter d to a tank inner diameter D is 0.01 or more and less than 1, and a ratio (h/H) of an agitating impeller height h to a tank height H of the reaction tank is 0.01 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<150> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, the pressure in the system in the mixing step (a2) is 0 MPaG or more and 10 MPaG or less, a peripheral speed of the agitating impeller is 0.05 m/s or more and 40 m/s or less when the reaction tank having the agitating impeller or the agitating impeller type mixer is used in the mixing step (a2), a ratio (d/D) of an impeller diameter d to a tank inner diameter D is 0.3 or more and less than 1, and a ratio (h/H) of an agitating impeller height h to a tank height H of the reaction tank is 0.3 or more and less than 1, and

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), a ratio (d/D) of an impeller diameter d to a tank inner diameter D is 0.01 or more and less than 1, and a ratio (h/H) of an agitating impeller height h to a tank height H of the reaction tank is 0.01 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio $(H_1/D_1)$ of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<151> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),

the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, the pressure in the system in the mixing step (a2) is 0 MPaG or more and 10 MPaG or less, a peripheral speed of the agitating impeller is 0.01 m/s or more and 50 m/s or less when the reaction tank having the agitating impeller or the agitating impeller type mixer is used in the mixing step (a2), and a ratio (d/D) of an impeller diameter d to a tank inner diameter D is 0.5 or more and less than 1,

the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), a ratio (d/D) of an agitating impeller diameter d to a tank inner diameter D is 0.01 or more and less than 1, and a ratio (h/H) of an agitating impeller height h to a tank height H of the reaction tank is 0.01 or more and less than 1, and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio $(H_1/D_1)$ of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<152> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
the mixing step (a2) is performed in a batch method, a semi-batch method, or a continuous method, the decolorizing agent is at least one selected from the group consisting of hydrogen peroxide, hypochlorous acid, nitric acid, potassium nitrate, permanganate, peroxide, chlorite, chlorate, perchlorate, and persulfate, the pressure in the system in the mixing step (a2) is 0 MPaG or more and 10 MPaG or less, a peripheral speed of the agitating impeller is 0.01 m/s or more and 50 m/s or less when the reaction tank having the agitating impeller or the agitating impeller type mixer is used in the mixing step (a2), and a ratio (h/H) of an agitating impeller height h to a tank height H of the reaction tank is 0.3 or more and less than 1,
the concentration of the alkali compound in the adjusting step (a1) is 0.005 mmol or more and 0.23 mmol or less with respect to 1 g of the internal olefin sulfonate after the mixing step (a2), the adjusting step (a1) is performed in a continuous method, a circulation ratio is greater than 0 times and 20 times or less, the pressure in the system in the adjusting step (a1) is 0 MPaG or more and 10 MPaG or less, a peripheral speed of the agitating impeller is 0.01 m/s or more and 50 m/s or less when the reaction tank having the agitating impeller or the agitating impeller type mixer is used in the adjusting step (a1), a ratio (d/D) of an impeller diameter d to a tank inner diameter D is 0.01 or more and less than 1, and a ratio (h/H) of an agitating impeller height h to a tank height H of the reaction tank is 0.01 or more and less than 1, and the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less,
a residence time in the aging step (b) is 10 minutes or more and 500 minutes or less, and a temperature range of the aging step (b) is 40°C or higher and 120°C or lower.

<153> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2),
the aging step (b) is performed in a continuous method, and a linear velocity of the internal olefin sulfonate composition is 0.001 mm/s or more and 1,000 mm/s or less.

<154> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and
the aging step (b) is performed in a continuous method, and a linear velocity of the internal olefin sulfonate composition is 0.1 mm/s or more and 1 mm/s or less.

<155> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed in a continuous method, and a linear velocity of the internal olefin sulfonate composition is 0.005 mm/s or more and 100 mm/s or less.

<156> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed using the vertical cylindrical reactor, and a ratio ($H_1/D_1$) of the height $H_1$ of the vertical cylindrical reactor to the inner diameter $D_1$ of the reactor is 0.1 or more and 300 or less.

<157> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed using the vertical cylindrical reactor, and a ratio ($H_1/D_1$) of the height $H_1$ of the vertical cylindrical reactor to the inner diameter $D_1$ of the reactor is 1.5 or more and 5 or less.

<158> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed using the vertical cylindrical reactor, and a ratio ($H_1/D_1$) of the height $H_1$ of the vertical cylindrical reactor to the inner diameter $D_1$ of the reactor is 1 or more and 50 or less.

<159> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and a pressure in the aging step (b) is 0 MPaG or more and 10 MPaG or less.

<160> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a pressure in the aging step (b) is 0.2 MPaG or more and 0.5 MPaG or less.

<161> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a pressure in the aging step (b) is 0.05 MPaG or more and 1 MPaG or less.

<162> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a residence time in the aging step (b) is 10 minutes or more and 500 minutes or less.

<163> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a residence time in the aging step (b) is 30 minutes or more and 200 minutes or less.

<164> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

a residence time in the aging step (b) is 20 minutes or more and 400 minutes or less.

<165> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and a temperature range of the aging step (b) is 40°C or higher and 120°C or lower.

<166> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and a temperature range of the aging step (b) is 60°C or higher and 100°C or lower.

<167> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and a temperature range of the aging step (b) is 50°C or higher and 110°C or lower.

<168> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed in a continuous method, a linear velocity of the internal olefin sulfonate composition is 0.001 mm/s or more and 1,000 mm/s or less, the aging step (b) is performed using the vertical cylindrical reactor, and a ratio ($H_1/D_1$) of the height $H_1$ of the vertical cylindrical reactor to the inner diameter $D_1$ of the reactor is 0.1 or more and 300 or less.

<169> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed in a continuous method, a linear velocity of the internal olefin sulfonate composition is 0.001 mm/s or more and 1,000 mm/s or less, the aging step (b) is performed using the vertical cylindrical reactor, and a ratio ($H_1/D_1$) of the height $H_1$ of the vertical cylindrical reactor to the inner diameter $D_1$ of the reactor is 1.5 or more and 5 or less.

<170> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and
the aging step (b) is performed in a continuous method, a linear velocity of the internal olefin sulfonate composition is 0.001 mm/s or more and 1,000 mm/s or less, the aging step (b) is performed using the vertical cylindrical reactor, and a ratio $(H_1/D_1)$ of the height $H_1$ of the vertical cylindrical reactor to the inner diameter $D_1$ of the reactor is 1 or more and 50 or less.

<171> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio $(H_1/D_1)$ of the height $H_1$ of the vertical cylindrical reactor to the inner diameter $D_1$ of the reactor is 0.1 or more and 300 or less, and a pressure in the aging step (b) is 0 MPaG or more and 10 MPaG or less.

<172> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio $(H_1/D_1)$ of the height $H_1$ of the vertical cylindrical reactor to the inner diameter $D_1$ of the reactor is 0.1 or more and 300 or less, and a pressure in the aging step (b) is 0.2 MPaG or more and 0.5 MPaG or less.

<173> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and
the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio $(H_1/D_1)$ of the height $H_1$ of the vertical cylindrical reactor to the inner diameter $D_1$ of the reactor is 0.1 or more and 300 or less, and a pressure in the aging step (b) is 0.05 MPaG or more and 1 MPaG or less.

<174> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, and a residence time in the aging step (b) is 10 minutes or more and 500 minutes or less.

<175> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, and a residence time in the aging step (b) is 60 minutes or more and 200 minutes or less.

<176> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, and a residence time in the aging step (b) is 20 minutes or more and 400 minutes or less.

<177> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and

the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 40°C or higher and 120°C or lower.

<178> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 60°C or higher and 100°C or lower.

<179> The method for producing a decolorized internal olefin sulfonate composition according to any one of <1> to <89>,

wherein the method includes a sulfonation process of sulfonating a raw material olefin, a neutralization process of neutralizing a sulfonated product obtained, a hydrolysis process of hydrolyzing a neutralization product obtained, and after the hydrolysis process, a decolorizing process (A) of performing an adjusting step (a1) of adjusting the concentration of an alkali compound in a system in which an internal olefin sulfonate composition containing an internal olefin sulfonate is present, a mixing step (a2) of mixing a decolorizing agent and the internal olefin sulfonate composition present in the system, and an aging step (b) after the mixing step (a2), and the aging step (b) is performed in a continuous method, a linear velocity of the internal olefin sulfonate composition is 0.001 mm/s or more and 1,000 mm/s or less, the aging step (b) is performed using the vertical cylindrical reactor, a ratio ($H_1/D_1$) of the height $H_1$ of the vertical cylindrical reactor to the inner diameter $D_1$ of the reactor is 0.1 or more and 300 or less, a pressure in the aging step (b) is 0 MPaG or more and 10 MPaG or less, a residence time in the aging step (b) is 10 minutes or more and 500 minutes or less, and a temperature range of the aging step (b) is 50°C or higher and 110°C or lower.

<180> Use of the decolorized internal olefin sulfonate composition obtained by the production method for producing a decolorized internal olefin sulfonate composition according to any one of <1> to <179> for at least one selected from the group consisting of body detergents, shampoos, clothing detergents, and tableware detergents.

EXAMPLES

Hereinafter, the present invention will be specifically described based on Examples. The contents of components in Tables are expressed in % by mass unless otherwise described. Various measurement and evaluation methods are as follows.

<Method for measuring double bond distribution of internal olefin>

A double bond position in an internal olefin was measured by gas chromatography (hereinafter, abbreviated as GC). Specifically, the internal olefin was caused to react with dimethyl sulfide to prepare a dithionated derivative thereof, and individual components of the resultant derivative were separated from each other by GC. From the individual peak areas, double bond positions of the internal olefin were determined. An average double bond position was calculated by the following formula. An apparatus used for the measurement and the analysis conditions are as follows.

[Apparatus and measurement conditions]

GC apparatus: HP6890 (manufactured by Hewlett-Packard Company)
Column: Ultra-Alloy-1HT capillary column, 30 m × 250 μm × 0.15 μm (manufactured by Frontier Laboratories Ltd.)
Detector: hydrogen flame ion detector (FID)
Injection temperature: 300°C
Detector temperature: 350°C
He flow rate: 4.6 mL/min

[Calculation formula of average double bond position]

[Math 1]

Case where n is odd (average double bond position) =

$$\sum_{m=1}^{m=(n-1)/2} m \times Y/100 \qquad (1)$$

Case where n is even (average double bond position) =

$$\sum_{m=1}^{m=n/2} m \times Y/100 \qquad (2)$$

(In the formula, n represents the number of carbon atoms of an olefin, m represents a double bond position in the olefin, and Y represents the percentage of a m-olefin in an olefin having n carbon atoms.)

<Method for producing internal olefin>

Production Example A (Synthesis of internal olefin having 16 carbon atoms)

Into a flask equipped with an agitator were charged 7,000 g (28.9 mol) of 1-hexadecanol "KALCOL 6098" (manufactured by Kao Corporation) and 700 g (10% by mass with respect to raw material alcohol) of γ-alumina (STREM Chemicals, Inc.) as a solid acid catalyst, and the mixture was reacted under agitating at 280°C for 3 hours or more with a flow of nitrogen (7,000 mL/min.) through the system. In order to obtain an internal olefin having a desired average double bond position, sampling was periodically performed to analyze the average double bond position, and cooling was started at an appropriate timing. The average double bond position after the completion of the reaction was 4.2 for the internal olefins used in Examples 1 to 14, 17, 20, 23, and 25 to 31, 4.3 for the internal olefin used in Example 15, 4.1 for the internal olefin used in Example 21, and 3.8 for Example 24.

Production Example B (Synthesis of internal olefin having 18 carbon atoms)

Into a flask equipped with an agitator were charged 7,000 g (25.9 mol) of 1-octadecanol "KALCOL 8098" (manufactured by Kao Corporation) and 1050 g (10% by mass with respect to raw material alcohol) of γ-alumina (STREM Chemicals, Inc.) as a solid acid catalyst, and the mixture was reacted under agitating at 280°C for 10 hours or more with a flow of nitrogen (7,000 mL/min.) through the system. In order to obtain an internal olefin having a desired average double bond position, sampling was periodically performed to analyze the average double bond position, and cooling was started at an appropriate timing. The average double bond position after the completion of the reaction was 4.2 for the internal olefin used in Example 16, 3.6 for the internal olefin used in Example 18, 4.4 for the internal olefin used in Example 22, 4.0 for the internal olefin used in Example 32, and 3.8 for Example 24.

<Method for producing decolorized internal olefin sulfonate composition>

Example 1

A thin-film type sulfonation reactor with an external jacket was used. Cooling water at 10°C was caused to flow through the external jacket, an internal olefin having 16 carbon atoms obtained in Production Example A was caused to flow down in the form of a thin film along the inner wall of the reactor, and a sulfonation reaction was performed using $SO_3$ gas ($SO_3$ concentration: 1.1 vol%) diluted with dehumidified air under the condition of $SO_3$/olefin molar ratio: 1.01 to obtain a sulfonated product.
A continuous loop neutralization reaction device equipped with Milder MDN303V (manufactured by Pacific Machinery & Engineering Co., Ltd.) as a mixer and having an external jacket was used. After ion-exchanged water was charged into the reaction device, the obtained sulfonated product and a potassium hydroxide aqueous solution (concentration: 17% by mass) were supplied under a condition that the temperature of a neutralization product at an outlet of the reaction device was 25°C to start a neutralization reaction. The peripheral speed of the mixer was 24 m/s. When the neutralization reaction device reached a steady state, the neutralization product was confirmed to flow out from the outlet of the reaction device, and the neutralization product was obtained. The concentration of an internal olefin sulfonate in the neutralization product was 55% by mass. The amount of an alkali compound used for neutralization was 1.04 times by mole with respect to a sulfonic acid group.

After the completion of the neutralization, the neutralization product was heated in an autoclave under a temperature condition of 160°C for 1 hour and hydrolyzed to obtain an internal olefin sulfonate composition before decolorizing treatment. The average double bond position of a raw material olefin of the internal olefin sulfonate was 4.2. The concentration of potassium hydroxide of the internal olefin sulfonate composition obtained by a hydrolysis treatment was 0.029 mmol with respect to 1 g of the internal olefin sulfonate.

A 48% potassium hydroxide aqueous solution was added to the internal olefin sulfonate composition, and the concentration of potassium hydroxide was adjusted to 0.246 mmol with respect to 1 g of the internal olefin sulfonate (adjusting step).

In a decolorizing process, a continuous loop decolorizing reaction device equipped with Milder MDN303V (manufactured by Pacific Machinery & Engineering Co., Ltd.) was used as a mixer. Milder MDN303V was equipped with G as a rotor and stator, and was operated in one stage. After the internal olefin sulfonate composition was heated to 80°C, the internal olefin sulfonate composition and a hydrogen peroxide solution (concentration: 45%) were supplied into the reaction device (mixing step), and a decolorizing treatment was started. The peripheral speed of the mixer in a mixing step was 4.7 m/s and the shear rate was 9,400/s. The amount of mixed hydrogen peroxide was 10 mg with respect to 1 g of the internal olefin sulfonate in the internal olefin sulfonate composition. The opening degree of a valve provided at the outlet of the reaction device was adjusted, to adjust a pressure in the reaction device to 0.3 MPaG. A residence time in the reaction device was 6 minutes. After the steady state, immediately after the mixing step, a pH meter installed in the decolorizing reaction device showed 11.4. The concentration of potassium hydroxide in the internal olefin sulfonate composition at this time was 0.233 mmol with respect to 1 g of the internal olefin sulfonate. The internal olefin sulfonate composition after the residence for 6 minutes was extracted, and held in a thermostatic chamber at the same temperature as that during the decolorizing treatment for 1 hour (aging step) to obtain the internal olefin sulfonate composition after decolorizing treatment. The internal olefin sulfonate composition after decolorizing treatment was subjected to measurement of hue, evaluation of a fading rate, evaluation of odor, evaluation of storage stability, and measurement of a peroxide value.

Examples 2 to 14 and 20 and Comparative Examples 1 and 2

Decolorized internal olefin sulfonate compositions were produced in the same manner as in Example 1 except that production conditions described in Tables 1 and 2 were employed. The concentration of potassium hydroxide was adjusted by adding a 48% potassium hydroxide aqueous solution or a 98% sulfuric acid aqueous solution to the internal olefin sulfonate composition before decolorizing treatment. Measurement of hue, evaluation of a fading rate, evaluation of odor, evaluation of storage stability, and measurement of a peroxide value were performed in the same manner as in Example 1. A concentration in an adjusting step in Table 1 indicates the concentration of an alkali compound immediately after a mixing step.

Example 15

An internal olefin sulfonate composition was obtained in the same manner as in Example 13 except that the internal olefin having 16 carbon atoms and an average double bond position of 4.3 was used among the internal olefins having 16 carbon atoms obtained in Production Example A. A decolorized internal olefin sulfonate composition was produced in the same manner as in Example 13 except that the obtained internal olefin sulfonate composition before decolorizing treatment was used. Measurement of hue, evaluation of a fading rate, evaluation of odor, evaluation of storage stability, and measurement of a peroxide value were performed in the same manner as in Example 13.

Example 16

An internal olefin sulfonate composition was obtained in the same manner as in Example 13 except that the internal olefin having 18 carbon atoms and an average double bond position of 4.2 obtained in Production Example B was used instead of the internal olefin having 16 carbon atoms obtained in Production Example A. A decolorized internal olefin sulfonate composition was produced in the same manner as in Example 13 except that the obtained internal olefin sulfonate composition before decolorizing treatment was used. Measurement of hue, evaluation of a fading rate, evaluation of odor, evaluation of storage stability, and measurement of a peroxide value were performed in the same manner as in Example 13.

Example 17

An internal olefin sulfonate composition before decolorizing treatment was produced in the same manner as in Example 13 except that a sodium hydroxide aqueous solution (concentration: 12.5% by mass) was used as an alkali agent instead of a potassium hydroxide aqueous solution. The concentration of an internal olefin sulfonate in a neutralization product was 53% by mass. An alkali concentration was adjusted by adding a 48% sodium hydroxide aqueous solution or a 98% sulfuric acid aqueous solution to the obtained internal olefin sulfonate composition before decolorizing treatment. The adjusted internal olefin sulfonate composition before decolorizing treatment was subjected to a decolorizing treatment in the same manner as in Example 13 except for production conditions described in Table 2. Thereafter, measurement of hue, evaluation of a fading rate, evaluation of odor, evaluation of storage stability, and measurement of a peroxide value were performed in the same manner as in Example 13.

Example 18

An internal olefin sulfonate composition before decolorizing treatment was obtained in the same manner as in Example 17 except that the internal olefin having 18 carbon atoms and an average double bond position of 3.6 obtained in Production Example B was used instead of the internal olefin having 16 carbon atoms obtained in Production Example A. The concentration of an internal olefin sulfonate in a neutralization product was 53% by mass. A decolorized internal olefin sulfonate composition was produced in the same manner as in Example 17 except for production conditions described in Table 2. Measurement of hue, evaluation of a fading rate, evaluation of odor, evaluation of storage stability, and measurement of a peroxide value were performed in the same manner as in Example 17.

Example 19

The storage stability of the decolorized internal olefin sulfonate composition obtained in Example 13 was evaluated in the same manner as in Example 13 except that the pH of the decolorized internal olefin sulfonate composition was adjusted using a phosphoric acid aqueous solution (concentration: 75% by mass). As a result, the storage stability was A as in Example 13.

Example 21

An internal olefin sulfonate composition was obtained in the same manner as in Example 13 except that the internal olefin having 16 carbon atoms and an average double bond position of 4.1 was used among the internal olefins having 16 carbon atoms obtained in Production Example A. A decolorized internal olefin sulfonate composition was produced in the same manner as in Example 13 except that the obtained internal olefin sulfonate composition before decolorizing treatment was used. Measurement of hue, evaluation of a fading rate, evaluation of odor, evaluation of storage stability, and measurement of a peroxide value were performed in the same manner as in Example 13.

Example 22

An internal olefin sulfonate composition was obtained in the same manner as in Example 16 except that the internal olefin having 18 carbon atoms and an average double bond position of 4.4 obtained in Production Example B was used instead of the internal olefin having 16 carbon atoms obtained in Production Example A. A decolorized internal olefin sulfonate composition was produced in the same manner as in Example 16 except that the obtained internal olefin sulfonate composition before decolorizing treatment was used. Measurement of hue, evaluation of a fading rate, evaluation of odor, evaluation of storage stability, and measurement of a peroxide value were performed in the same manner as in Example 16.

Example 23

An internal olefin sulfonate composition was obtained by the method of Example 1 using the internal olefin having 16 carbon atoms obtained in Production Example A. 98% sulfuric acid was added to the obtained internal olefin sulfonate composition before decolorizing treatment to adjust the concentration of potassium hydroxide. The concentration of potassium hydroxide was 0.054 mmol with respect to 1 g of an internal olefinate. A decolorized internal olefin sulfonate composition was produced in the same manner as in Example 13 except that an adjusting step and a mixing step were performed in parallel on the adjusted internal olefin sulfonate composition before decolorizing treatment (circulation ratio in the mixing step and the adjusting step: 4 times). In the adjusting step, a 48% potassium hydroxide aqueous solution was added. The concentration of potassium hydroxide immediately after the mixing step was 0.059 mmol with respect to 1 g of an internal olefin sulfonate. Thereafter, measurement of hue, evaluation of a fading rate, evaluation of odor, evaluation of storage stability, and measurement of a peroxide value were performed in the same manner as in Example 13.

<Measurement and Evaluation Methods>

(Measurement of potassium hydroxide (or sodium hydroxide) concentration in internal olefin sulfonate composition)

An appropriate amount (about 5 g with a precision of three digits after the decimal point) of a sample was weighed and diluted with ion-exchanged water, and then about 0.05 g of a 1% phenolphthalein ethanol solution was added to the diluted solution to develop a red color. The sample solution was titrated with 1/10 N hydrochloric acid, and the titer when the red color no longer developed red was read. The concentration of potassium hydroxide (or sodium hydroxide) in the sample was determined by the following formula.

$$\text{Concentration of potassium hydroxide (or sodium hydroxide) (mmol/internal olefin sulfonate } 1g) = \{(1/10 \times f \times d \times M)/(c \times 1{,}000)\} \times 100$$

c: Sampling amount (g)
d: Amount of 1/10 N hydrochloric acid used (ml)
f: Factor of 1/10N hydrochloric acid
M: Molecular weight of potassium hydroxide (or sodium hydroxide)

Regarding a region (pH of less than 8.3) in which phenolphthalein does not color, the concentration of potassium hydroxide (or sodium hydroxide) in the sample was determined by the following method. When the concentration of potassium hydroxide (or sodium hydroxide) in the sample is plotted on the abscissa and the pH is plotted on the ordinate, the concentration of potassium hydroxide (or sodium hydroxide) in the sample was calculated from the measured pH value according to the following formula, which was established by considering these relationships from pH 7 to 8.3 as approximately linear relationships.

Concentration of potassium hydroxide (or sodium hydroxide) (mmol/internal olefin sulfonate 1g)=((measured pH)-7)/1010.1

(Measurement of hue and evaluation of fading rate)

A tristimulus value direct reading type petroleum product color tester "OME 2000" (manufactured by Nippon Denshoku Industries Co., Ltd.) was used. The Hazen color number (APHA) of each of the internal olefin sulfonate composition before decolorizing treatment and the decolorized internal olefin sulfonate composition after decolorizing treatment at 25°C was measured. Before the measurement, the concentration of the internal olefin sulfonate in each composition was adjusted to 10% by mass.
When the Hazen color number before decolorizing treatment was A and the Hazen color number after decolorizing treatment was B, the fading rate (%) was calculated according to the following calculation formula. The larger the absolute value of the fading rate was, the more excellent the decolorizing effect was, and evaluation was performed according to the following criteria.

$$\text{Fading rate } (\%) = 100 \times (A-B)/A$$

[Evaluation criteria]

A: Fading rate of 70% or more
B: Fading rate of 65% or more and less than 70%
C: Fading rate of 60% or more and less than 65%
D: Fading rate of less than 60%

(Evaluation of odor)

The odor level of the internal olefin sulfonate composition after decolorizing treatment produced in each of Examples 1 to 23 and Comparative Example 2 was compared with that of a standard product (the internal olefin sulfonate composition after decolorizing treatment produced in Comparative Example 1). Five odor evaluation experts evaluated each of the odor levels according to the following criteria. Then, the evaluation scores of the odor levels evaluated by the five experts were averaged, and comprehensive evaluations (A to C) were defined as follows.

[Evaluation criteria]

3 points: Much lower than the odor level of the standard product
2 points: Slightly lower than the odor level of the standard product
1 point: Equal to the odor level of the standard product

[Comprehensive evaluation]

A: Average of 2.5 points or more
B: Average of 1.5 points or more and less than 2.5 points
C: Average of less than 1.5 points

(Evaluation of storage stability)

$K_2SO_4$ monohydrate was added to the produced internal olefin sulfonate composition after decolorizing treatment so that the concentration of $K_2SO_4$ was 5 parts by mass with respect to 100 parts by mass of the internal olefin sulfonate. Thereafter, a 50% citric acid aqueous solution (in Example 19, a 75% phosphoric acid aqueous solution was used) was added to the internal olefin sulfonate composition, and a pH at 25°C when the concentration of the internal olefin sulfonate in the internal olefin sulfonate composition after decolorizing treatment was adjusted to 10% by mass was adjusted to 10 (pH adjusting step). Thereafter, ion-exchanged water was added to the decolorized internal olefin sulfonate composition adjusted above, and the concentration of the internal olefin sulfonate of the internal olefin sulfonate composition was adjusted to 25%. After the adjustment, the internal olefin sulfonate composition was stored at 25°C for 24 hours. Thereafter, the storage stability was evaluated. The evaluation was performed visually and performed according to the following criteria.

A: No precipitates are formed.
B: A small amount of precipitates is formed and dispersed.
C: A large amount of precipitates is formed and deposited.

(Measurement of peroxide value)

10 g of the produced decolorized internal olefin sulfonate composition was placed in a stoppered Erlenmeyer flask, 15 mL of chloroform and 20 mL of acetic acid were added thereto, and the flask was stoppered to dissolve the decolorized internal olefin sulfonate composition. Air in the stoppered Erlenmeyer flask was replaced with nitrogen gas. 1 mL of a saturated aqueous solution of potassium iodide was added while the nitrogen gas was passed therethrough, the Erlenmeyer flask was stoppered and shaken for 1 minute, and then the Erlenmeyer flask was stored as it was in a normal temperature dark room for 5 minutes. Thereafter, 75 mL of a methyl alcohol aqueous solution obtained by mixing methyl alcohol and water at a ratio of 1:1 in advance was added into the Erlenmeyer flask, the Erlenmeyer flask was stoppered again for mixing. Then, the stopper was washed with about 50 mL of a methyl alcohol aqueous solution, and immediately, 0.01 mol/L sodium thiosulfate aqueous solution titration was performed using a potentiometric titrator (AT-510 manufactured by Kyoto Electronics Manufacturing Co., Ltd.). At the same time, a blank test (no addition of decolorized internal olefin sulfonate composition) was performed. In Table, the amounts of peroxide values with respect to 1 kg of the internal olefin sulfonate in the internal olefin sulfonate composition were expressed, and the results evaluated according to the following evaluation criteria were also shown.

$$\text{Peroxide value (meq/kg)} = (a-b) \times f \times 10 / \text{sampling amount (g)}$$

a: Used amount (mL) of 0.01 mol/L sodium thiosulfate aqueous solution required for titration of sample
b: Used amount (mL) of 0.01 mol/L sodium thiosulfate aqueous solution required for titration in blank test
f: Factor of 0.01 mol/L sodium thiosulfate aqueous solution

[Evaluation criteria]

A: 11.5 meq/kg or less
B: greater than 11.5 meq/kg and 27 meq/kg or less
C: greater than 27 meq/kg

(Method for measuring pH when concentration of internal olefin sulfonate is 10% by mass)

Measurement was performed using a pH meter (Horiba, Ltd., pH meter D-51) calibrated with a pH standard solution. The decolorized internal olefin sulfonate composition was diluted with ion-exchanged water so that the concentration of the internal olefin sulfonate was 10% by mass, and the obtained diluted solution was adjusted to 25°C. Then, a pH glass electrode of the pH meter was then immersed in the diluted solution, and a constantly displayed value was read.

[Table 1]

| | | Comparative Example 1 | Comparative Example 2 | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|
| INTERNAL OLEFIN SULFONATE (IOS) | CARBON CHAIN LENGTH | C16 | C16 | C16 | C16 | C16 | C16 | C16 | C16 | C16 | C16 | C16 | C16 |
| | AVERAGE DOUBLE BOND POSITION OF RAW MATERIAL OLEFIN | 4.2 | 4.2 | 4.2 | 4.2 | 4.2 | 4.2 | 4.2 | 4.2 | 4.2 | 4.2 | 4.2 | 4.2 |

(continued)

| DECOLORING TREATING PROCESS | | | | | Comparative Example 1 | Comparative Example 2 | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|---|---|---|
| | ADJUSTING STEP | CONCENTRATION OF ALKALI COMPOUND WITH RESPECT TO 1 g OF IOS | KIND | | KOH | KOH | KOH | KOH | KOH | KOH | KOH | KOH | KOH | KOH | KOH | KOH |
| | | | [mmol] | | 0.002 | 0.287 | 0.233 | 0.198 | 0.059 | 0.005 | 0.059 | 0.059 | 0.059 | 0.047 | 0.047 | 0.047 |
| | | ADDITION AMOUNT OF $H_2O_2$ WITH RESPECT TO 1 g OF IOS | [mg] | | 10 | 10 | 10 | 10 | 10 | 10 | 5 | 20 | 40 | 10 | 10 | 10 |
| | MIXING STEP | PERIPHERAL SPEED OF AGITATING IMPELLER | [m/s] | | 4.7 | 4.7 | 4.7 | 4.7 | 4.7 | 4.7 | 4.7 | 4.7 | 4.7 | 0.9 | 2.4 | 23.6 |
| | | SHEAR RATE | [1/s] | | 9400 | 9400 | 9400 | 9400 | 9400 | 9400 | 9400 | 9400 | 9400 | 1800 | 4800 | 47200 |
| | | TEMPERATURE | [°C] | | 80 | 80 | 80 | 80 | 80 | 80 | 80 | 80 | 80 | 80 | 80 | 80 |
| | | pH | [-] | | 9.2 | 11.5 | 11.4 | 11.3 | 10.7 | 9.8 | 10.8 | 10.2 | 9.8 | 10.4 | 10.4 | 10.4 |
| | AGING STEP | AGING REACTOR | [ - ] | | THERMOSTATIC BATH | THERMOSTATIC BATH | THERMOSTATIC BATH | THERMOSTATIC BATH | THERMOSTATIC BATH | THERMOSTATIC BATH | THERMOSTATIC BATH | THERMOSTATIC BATH | THERMOSTATIC BATH | THERMOSTATIC BATH | THERMOSTATIC BATH | THERMOSTATIC BATH |
| | | TEMPERATURE | [°C] | | 80 | 80 | 80 | 80 | 80 | 80 | 80 | 80 | 80 | 80 | 80 | 80 |
| | | RESIDENCE TIME | [min] | | 60.0 | 60.0 | 60.0 | 60.0 | 60.0 | 60.0 | 60.0 | 60.0 | 60.0 | 60.0 | 60.0 | 60.0 |

(continued)

| | | | Comparative Example 1 | Comparative Example 2 | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|---|
| EVALUATION | EVALUATION OF ODOR | [-] | C | A | A | A | A | A | A | A | A | A | A | A |
| | EVALUATION OF STORAGE STABILITY | [-] | - | C | B | A | A | A | A | A | A | A | A | A |
| | FADING RATE | [%] | 76.0 | 60.6 | 56.9 | 64.9 | 65.9 | 70.8 | 63.1 | 65.9 | 74.1 | 60.1 | 67.2 | 69.2 |
| | EVALUATION OF FADING RATE | [-] | A | C | D | C | B | A | C | B | A | C | B | B |
| | MEASUREMENT OF PEROXIDE VALUE (WITH RESPECT TO 1 kg OF IOS) | [meq/k g] | - | 2.2 | 2.6 | 2.8 | 3.3 | 5.7 | 2.7 | 4.0 | 7.3 | 5.2 | 4.4 | 4.4 |
| | EVALUATION OF PEROXIDE VALUE | [-] | - | A | A | A | A | A | A | A | A | A | A | A |

[Table 2]

| INTERNAL OLEFIN SULFONATE (IOS) | | Example 11 | Example 12 | Example 13 | Example 14 | Example 15 | Example 16 | Example 17 | Example 18 | Example 20 | Example 21 | Example 22 | Example 23 |
|---|---|---|---|---|---|---|---|---|---|---|---|---|---|
| | CARBON CHAIN LENGTH | C16 | C16 | C16 | C16 | C16 | C18 | C16 | C18 | C16 | C16 | C18 | C16 |
| | AVERAGE DOUBLE BOND POSITION OF RAW MATERIAL OLEFIN | 4.2 | 4.2 | 4.2 | 4.2 | 4.3 | 4.2 | 4.2 | 3.6 | 4.2 | 4.1 | 4.4 | 4.2 |

(continued)

| | | | | Example 11 | Example 12 | Example 13 | Example 14 | Example 15 | Example 16 | Example 17 | Example 18 | Example 20 | Example 21 | Example 22 | Example 23 |
|---|---|---|---|---|---|---|---|---|---|---|---|---|---|---|---|
| DECO-LOR ING TREATIN G PRO-CESS | AD-JUST ING STEP | CON-CENTR ATION OF ALKALI COM-POUND WITH RE-SPECT TO 1 g OF IOS | KIND | KOH | KOH | KOH | KOH | KOH | KOH | NaOH | NaOH | KOH | KOH | KOH | KOH |
| | | | [mmol] | 0.059 | 0.059 | 0.059 | 0.059 | 0.059 | 0.059 | 0.068 | 0.096 | 0.004 | 0.059 | 0.059 | 0.059 |
| | MIXING STEP | ADDITION AMOUNT OF $H_2O_2$ WITH RE-SPECT TO 1 g OF IOS | [mg] | 20 | 20 | 20 | 20 | 20 | 20 | 20 | 20 | 10 | 20 | 20 | 20 |
| | | PERIPHER AL SPEED OF AGITA-TIN G IM-PELLER | [m/s] | 9.4 | 9.4 | 9.4 | 9.4 | 9.4 | 9.4 | 9.4 | 9.4 | 4.7 | 9.4 | 9.4 | 9.4 |
| | | SHEAR RATE | [1/s] | 18800 | 18800 | 18800 | 18800 | 18800 | 18800 | 18800 | 18800 | 9400 | 18800 | 18800 | 18800 |
| | | TEMPERAT URE | [°C] | 60 | 70 | 80 | 90 | 80 | 80 | 80 | 80 | 80 | 80 | 80 | 80 |
| | | pH | [-] | 10.2 | 10.2 | 10.2 | 10.2 | 10.2 | 10.2 | 10.2 | 10.3 | 9.5 | 10.2 | 10.2 | 10.2 |
| | AGING STEP | AGING RE-ACTOR | [-] | THER-MOST ATIC BATH | THER-MOST ATIC BATH | THER-MOST ATIC BATH | THER-MOST ATIC BATH | THER-MOST ATIC BATH | THER-MOST ATIC BATH | THER-MOST ATIC BATH | THER-MOST ATIC BATH | THER-MOST ATIC BATH | THER-MOST ATIC BATH | THER-MOST ATIC BATH | THER-MOST ATIC BATH |
| | | TEMPERAT URE | [°C] | 60 | 70 | 80 | 90 | 80 | 80 | 80 | 80 | 80 | 80 | 80 | 80 |
| | | RESIDENC E TIME | [min] | 60.0 | 60.0 | 60.0 | 60.0 | 60.0 | 60.0 | 60.0 | 60.0 | 60.0 | 60.0 | 60.0 | 60.0 |

(continued)

| | | Example 11 | Example 12 | Example 13 | Example 14 | Example 15 | Example 16 | Example 17 | Example 18 | Example 20 | Example 21 | Example 22 | Example 23 |
|---|---|---|---|---|---|---|---|---|---|---|---|---|---|
| EVALUATION | EVALUATION OF ODOR [-] | A | A | A | A | A | A | A | A | B | A | A | A |
| | EVALUATION OF STORAGE STABILITY [-] | A | A | A | A | A | A | A | A | A | A | A | A |
| | FADING RATE [%] | 56.7 | 65.9 | 73.0 | 79.9 | 77.6 | 70.9 | 78.1 | 69.8 | 69.2 | 80.0 | 68.4 | 76.4 |
| | EVALUATION OF FADING RATE [-] | D | B | A | A | A | A | A | B | B | A | B | A |
| | MEASUREMENT OF PEROXIDE VALUE (WITH RESPECT TO 1 kg OF IOS) [meq/k g] | 35.6 | 5.3 | 4.3 | 3.3 | 4.6 | 4.5 | 5.5 | 4.5 | 6.8 | 6.2 | 5.4 | 3.9 |
| | EVALUATION OF PEROXIDE VALUE [-] | C | A | A | A | A | A | A | A | A | A | A | A |

In Examples 1 to 23, by the adjusting step of the decolorizing process, the concentration of potassium hydroxide or sodium hydroxide in the internal olefin sulfonate composition at the start of the decolorizing treatment was adjusted to 0.004 mmol or more and 0.26 mmol or less with respect to 1 g of the internal olefin sulfonate, so that the decolorized internal olefin sulfonate composition having a good color fading effect, a low odor level, and good storage stability was obtained. Meanwhile, in Comparative Examples 1 and 2, the odor of the internal olefin sulfonate composition after decolorizing treatment became strong, or the storage stability was deteriorated.

Example 24

An internal olefin sulfonate composition before decolorizing treatment was obtained in the same manner as in Example 1 using an internal olefin mixture (C16/C18 mass ratio : 80/20) obtained by mixing the internal olefin having 16 carbon atoms and an average double bond position of 3.8 obtained in Production Example A and the internal olefin having 18 carbon atoms and an average double bond position of 3.8 obtained in Production Example B. The average double bond position of a raw material olefin of the internal olefin sulfonate was 3.8. Sodium hydroxide was used as an alkali agent used for neutralization. The internal olefin sulfonate composition obtained by a hydrolysis treatment had a sodium hydroxide concentration of 0.08 mmol with respect to 1 g of the internal olefin sulfonate, and an effective component concentration of 65% by mass.

The obtained internal olefin sulfonate composition before decolorizing treatment was charged in an amount of 180 kg into a reaction tank (volume: about 250 L). The reaction tank was equipped with MR205 (manufactured by Satake MultiMix Corporation) as an agitating impeller, where impeller diameter d/reaction tank inner diameter D was 0.59, and agitating impeller height h/reaction tank height H was 0.61. A circulation line was provided outside the reaction tank, Milder MDN303V (manufactured by Pacific Machinery & Engineering Co., Ltd.) was provided as a mixer in the circulation line. The Milder MDN303V was equipped with G as a rotor and stator, and was operated in one stage.

The internal olefin sulfonate composition was charged into the device, and heated to 80°C. Then, the internal olefin sulfonate composition in the reaction tank was supplied to the Milder section. Then, a 48% sodium hydroxide aqueous solution and a hydrogen peroxide solution (concentration: 35%) were simultaneously supplied, and a decolorizing treatment was started. The hydrogen peroxide solution was added dropwise in 20 portions over 2 hours to start the decolorizing treatment (semi-batch method) (mixing step and adjusting step). The amount of mixed hydrogen peroxide was 10 mg with respect to 1 g of the internal olefin sulfonate in the internal olefin sulfonate composition. The peripheral speed in the mixer in the mixing step and the adjusting step was 0.9 m/s, and the shear rate was 1,800/s. The pressure in the reaction tank was normal pressure (0 MPaG). In the mixing step, a pH meter installed in the decolorizing reaction device showed 11.0±0.2. The concentration of sodium hydroxide in the internal olefin sulfonate composition immediately after the mixing step was 0.08 mmol with respect to 1 g of the internal olefin sulfonate. Thereafter, the internal olefinate composition after decolorizing treatment in the reaction device was aged at 80°C, normal pressure (0 MPaG), and a residence time of 60 minutes (aging step). The internal olefin sulfonate composition after the residence for 60 minutes was extracted to obtain the internal olefin sulfonate composition after decolorizing treatment. The internal olefin sulfonate composition after decolorizing treatment was subjected to measurement of hue, evaluation of a fading rate, evaluation of odor, evaluation of storage stability, and measurement of a peroxide value.

Example 25

The internal olefin sulfonate composition before decolorizing treatment obtained in Example 1 was charged in an amount of 180 kg into a reaction tank (volume: about 250 L) equipped with an agitating impeller. The reaction tank was equipped with MR205 (manufactured by Satake MultiMix Corporation) as an agitating impeller, where impeller diameter d/reaction tank inner diameter D was 0.59, and agitating impeller height h/reaction tank height H was 0.61. The internal olefin sulfonate composition was charged into the reaction tank (batch method), and heated to 80°C. Then, a 48% potassium hydroxide aqueous solution was added dropwise to adjust an alkali concentration to 0.041 mmol. Thereafter, a hydrogen peroxide solution (concentration: 35%) was added dropwise into the reaction tank in five portions over 2 hours, a 48% sodium hydroxide aqueous solution was simultaneously supplied (mixing step and adjusting step), and a decolorizing treatment was started. The peripheral speed of the reaction impeller in the mixing step and the adjusting step was 1.74 m/s, the shear rate was 13.41/s, and stirring power per unit liquid amount (Pv) per unit liquid amount was 0.453 kW/m$^3$. The pressure in the reaction tank was normal pressure (0 MPaG). The amount of mixed hydrogen peroxide was 20 mg with respect to 1 g of the internal olefin sulfonate in the internal olefin sulfonate composition. In the mixing step, a pH meter installed in the decolorizing reaction tank showed 9.6±0.3. The concentration of potassium hydroxide in the internal olefin sulfonate composition immediately after the mixing step was 0.041 mmol with respect to 1 g of the internal olefin sulfonate. Thereafter, the internal olefin sulfonate composition after decolorizing treatment in the reaction tank was aged at 80°C, normal pressure (0 MPaG), and a residence time of 30 minutes (aging step). The internal olefin sulfonate composition after the residence for 30 minutes was extracted to obtain the internal olefin sulfonate composition after decolorizing treatment.

The internal olefin sulfonate composition after decolorizing treatment was subjected to measurement of hue, evaluation of a fading rate, evaluation of odor, evaluation of storage stability, and measurement of a peroxide value.

Example 26

A decolorized internal olefin sulfonate composition was produced in the same manner as in Example 25 except that production conditions described in Table 3 were employed. Measurement of hue, evaluation of a fading rate, evaluation of odor, evaluation of storage stability, and measurement of a peroxide value were performed in the same manner as in Example 1. A concentration in an adjusting step in Table 3 indicates the concentration of an alkali compound immediately after a mixing step. The concentration of the alkali compound in the internal olefin sulfonate composition before the mixing step was 0.06 mmol. In the mixing step, a pH meter installed in the decolorizing reaction tank showed 10.4±0.2.

[Table 3]

| | | | | Example 24 | Example 25 | Example 26 |
|---|---|---|---|---|---|---|
| INTERNAL OLEFIN SULFONATE (IOS) | | | CARBON CHAIN LENGTH | C16/C18 | C16 | C16 |
| | | | AVERAGE DOUBLE BOND PO-SITION OF RAW MA-TERIAL OLEFIN | 3.8 | 4.2 | 4.2 |
| DECOLORING TREATING PROCESS | ADJUSTING STEP | CONCENTRATION OF ALKALI COM-POUND WITH RE-SPECT TO 1 g OF IOS | KIND | NaOH | KOH | KOH |
| | | | [mmol] | 0.08 | 0.041 | 0.06 |
| | | PERIPHERAL SPEED OF AGITATING IMPELLER | [m/s] | 0.9 | 1.74 | 1.96 |
| | | SHEAR RATE | [1/s] | 1800 | 13.41 | 15.05 |
| | | STIRRING POWER PER UNIT LIQUID AMOUNT Pv | [kW/m$^3$] | - | 0.453 | 0.53 |
| | MIXING STEP | ADDITION AMOUNT OF H$_2$O$_2$ WITH RESPECT TO 1 g OF IOS | [mg] | 10 (20 POR-TIONS) | 20 (5 POR-TIONS) | 6 (6 POR-TIONS) |
| | | PERIPHERAL SPEED OF AGITAT-ING IMPELLER | [m/s] | 0.9 | 1.74 | 1.96 |
| | | SHEAR RATE | [1/s] | 1800 | 13.41 | 15.05 |
| | | STIRRING POWER PER UNIT LIQUID AMOUNT Pv | [kW/m$^3$] | - | 0.453 | 0.53 |
| | | TEMPERATURE | [°C] | 80 | 80 | 80 |
| | | RESIDENCE TIME | [min] | 60 | 30 | 60 |
| | | pH | [-] | 11.0 | 9.6 | 10.4 |

(continued)

|  |  |  | Example 24 | Example 25 | Example 26 |
|---|---|---|---|---|---|
| EVALUATION | EVALUATION OF ODOR | [-] J | A | A | A |
| | EVALUATION OF STORAGE STABILITY | [-] | A | A | A |
| | FADING RATE | [%] | 75.8 | 53.9 | 55.5 |
| | EVALUATION OF FADING RATE | [-] | A | D | D |
| | MEASUREMENT OF PEROXIDE VALUE (WITH RESPECT TO 1 kg OF IOS) | [meq/kg] | 7.4 | 5.3 | 6.9 |
| | EVALUATION OF PEROXIDE VALUE | [-] | A | A | A |

In Example 24, the decolorizing process was performed in a semi-batch method, and the decolorized internal olefin sulfonate composition having a good color fading effect, a low odor level, and good storage stability was obtained. In Examples 25 and 26, the decolorizing process was performed in a batch method, and the decolorized internal olefin sulfonate composition having a low odor level and good storage stability could be obtained.

Example 27

An internal olefin sulfonate composition before decolorizing treatment was obtained in the same manner as in Example 1. The average double bond position of a raw material olefin of the internal olefin sulfonate was 4.2. The concentration of potassium hydroxide of the internal olefin sulfonate composition obtained by a hydrolysis treatment was 0.029 mmol with respect to 1 g of the internal olefin sulfonate.

A 48% potassium hydroxide aqueous solution was added to the internal olefin sulfonate composition, and the concentration of potassium hydroxide was adjusted to 0.030 mmol with respect to 1 g of the internal olefin sulfonate immediately after the mixing step (adjusting step). The concentration of potassium hydroxide after the adjustment was 0.043 mmol with respect to 1 g of the internal olefin sulfonate.

In a decolorizing process, a continuous loop decolorizing reaction device equipped with Milder MDN303V (manufactured by Pacific Machinery & Engineering Co., Ltd.) was used as a mixer. Milder MDN303V was equipped with G as a rotor and stator, and was operated in one stage. After the internal olefin sulfonate composition was heated to 80°C, the internal olefin sulfonate composition and a hydrogen peroxide solution (concentration: 45%) were supplied into the reaction device, the opening degree of a valve provided at the outlet of the reaction device was adjusted, the pressure in the reaction device was adjusted to 0.3 MPaG, and a decolorizing treatment was started (mixing step). The peripheral speed of the mixer in a mixing step was 4.7 m/s and the shear rate was 9,400/s. The amount of mixed hydrogen peroxide was 10 mg with respect to 1 g of the internal olefin sulfonate in the internal olefin sulfonate composition. After the steady state, immediately after the mixing step, a pH meter installed in the decolorizing reaction device showed 12. The concentration of potassium hydroxide in the internal olefin sulfonate composition at this time was 0.030 mmol with respect to 1 g of the internal olefin sulfonate. A residence time in the reaction device was 40 minutes. The internal olefin sulfonate composition after the residence for 40 minutes was extracted to obtain the internal olefin sulfonate composition after decolorizing treatment. The internal olefin sulfonate composition after decolorizing treatment was subjected to measurement of hue, evaluation of a fading rate, evaluation of odor, evaluation of storage stability, and measurement of a peroxide value. A concentration in the adjusting step in Table 4 indicates the concentration of an alkali compound immediately after the mixing step.

Example 28

A decolorized internal olefin sulfonate composition was produced in the same manner as in Example 27 except that production conditions described in Table 4 were employed. The obtained decolorized internal olefin sulfonate composition was supplied from the lower part of a vertical cylindrical reactor in which the ratio ($H_1/D_1$) of a height ($H_1$) to a tank inner diameter ($D_1$) was 2.9 to the reactor, and an aging step was performed at a temperature of 80°C, a pressure of 0.30 MPaG, a residence time of 34 minutes and a linear velocity of 0.3 mm/s. After the steady state, the decolorized internal olefin sulfonate composition after aging was obtained from the outlet of the upper part of the vertical cylindrical reactor, and measurement of hue, evaluation of a fading rate, evaluation of odor, evaluation of storage stability, and measurement of a peroxide value were performed in the same manner as in Example 1. A concentration in the adjusting step in Table 4 indicates the concentration of an alkali compound immediately after the mixing step.

Examples 29 to 32

A decolorized internal olefin sulfonate composition was produced in the same manner as in Example 28 except that production conditions described in Table 4 were employed. In Examples 31 and 32, an adjusting step and a mixing step were performed in parallel, and a circulation ratio at that time was set to 4 times. An alkali concentration was adjusted by adding a 48% potassium hydroxide aqueous solution. The obtained decolorized internal olefin sulfonate composition after aging was subjected to measurement of hue, evaluation of a fading rate, evaluation of odor, evaluation of storage stability, and measurement of a peroxide value in the same manner as in Example 1. A concentration in the adjusting step in Table 4 indicates the concentration of an alkali compound immediately after the mixing step.

[Table 4]

| | | Example 27 | Example 28 | Example 29 | Example 30 | Example 31 | Example 32 |
|---|---|---|---|---|---|---|---|
| INTERNAL OLEFIN SULFONATE (IOS) | CARBON CHAIN LENGTH | C16 | C16 | C16 | C16 | C16 | C18 |
| | AVERAGE DOUBLE BOND POSITION OF RAW MATERIAL OLEFIN | 4.2 | 4.2 | 4.2 | 4.2 | 4.2 | 4.0 |

(continued)

| | | | | Example 27 | Example 28 | Example 29 | Example 30 | Example 31 | Example 32 |
|---|---|---|---|---|---|---|---|---|---|
| DECOLORING TREATING PROCESS | ADJUSTING STEP | CONCENTRATION OF ALKALI COMPOUND WITH RESPECT TO 1 g OF IOS | KIND | KOH | KOH | KOH | KOH | KOH | KOH |
| | | | [mmol] | 0.030 | 0.030 | 0.048 | 0.048 | 0.053 | 0.053 |
| | MIXING STEP | ADDITION AMOUNT OF $H_2O_2$ WITH RESPECT TO 1 g OF IOS | [mg] | 10 | 10 | 20 | 20 | 20 | 18 |
| | | PERIPHERAL SPEED OF AGITATING IMPELLER | [m/s] | 4.7 | 4.7 | 4.7 | 4.7 | 9.4 | 9.4 |
| | | SHEAR RATE | [1/s] | 9400 | 9400 | 9400 | 9400 | 18800 | 18800 |
| | | TEMPERATURE | [°C] | 80 | 80 | 80 | 80 | 80 | 80 |
| | | pH | [-] | 11.0 | 11.0 | 10.9 | 10.9 | 10.8 | 11.0 |
| | | PRESSURE | [MPaG] | 0.30 | 0.30 | 0.35 | 0.35 | 0.35 | 0.35 |
| | | RESIDENCE TIME | [min] | 40.0 | 6.0 | 6.0 | 6.0 | 1.0 | 1.0 |
| | AGING STEP | AGING REACTOR | [-] | | VERTICAL CYLINDRICAL | VERTICAL CYLINDRICAL | VERTICAL CYLINDRICAL | VERTICAL CYLINDRICAL | VERTICAL CYLINDRICAL |
| | | H/D | [-] | | 2.9 | 3.8 | 3.8 | 1.5 | 1.5 |
| | | TEMPERATURE | [°C] | | 80 | 80 | 80 | 80 | 80 |
| | | PRESSURE | [MPaG] | | 0.30 | 0.10 | 0.30 | 0.30 | 0.30 |
| | | RESIDENCE TIME | [min] | | 34.0 | 25.0 | 25.0 | 90.0 | 100.0 |
| | | LINEAR VELOCITY | [mm/s] | | 0.3 | 0.1 | 0.1 | 0.3 | 0.3 |

(continued)

| EVALUATION | | | | Example 27 | Example 28 | Example 29 | Example 30 | Example 31 | Example 32 |
|---|---|---|---|---|---|---|---|---|---|
| | EVALUATION OF ODOR | | [-] | A | A | A | A | A | A |
| | EVALUATION OF STORAGE STABILITY | | [-] | A | A | A | A | A | A |
| | FADING RATE | | [%] | 74.2 | 76.9 | 65.5 | 65.5 | 76.2 | 77.9 |
| | EVALUATION OF FADING RATE | | [-] | A | A | B | B | A | A |
| | MEASUREMENT OF PEROXIDE VALUE (WITH RESPECT TO 1 kg OF IOS) | | [meq/kg] | 23.1 | 10.4 | 11.8 | 4.1 | 9.6 | 11.2 |
| | EVALUATION OF PEROXIDE VALUE | | [-] | B | A | B | A | A | A |

In Examples 27 to 32, the decolorized internal olefin sulfonate compositions having a good color fading effect, a low odor level, and good storage stability were obtained. Furthermore, in Examples 28 to 32, the decolorized internal olefin sulfonate compositions having a further reduced peroxide value were obtained by providing the aging step after the decolorizing process.

INDUSTRIAL APPLICABILITY

The decolorized internal olefin sulfonate composition obtained by the production method of the present invention is useful as a base for various detergents.

**Claims**

1. A method for producing a decolorized internal olefin sulfonate composition comprising a decolorizing process (A) of performing an adjusting step (a1) of adjusting a concentration of an alkali compound in a system in which an internal olefin sulfonate composition containing an internal olefin sulfonate is present and a mixing step (a2) of mixing a decolorizing agent and the internal olefin sulfonate composition present in the system,
wherein the adjusting step (a1) is a step of adjusting the concentration of the alkali compound in the decolorizing process (A) so that the concentration of the alkali compound after mixing the decolorizing agent and the internal olefin sulfonate composition present in the system is 0.004 mmol or more and 0.26 mmol or less with respect to 1 g of the internal olefin sulfonate.

2. The method according to claim 1, wherein the adjusting step (a1) is performed after the mixing step (a2) is performed.

3. The method according to claim 1, wherein the mixing step (a2) is performed after the adjusting step (a1) is performed.

4. The method according to claim 1, wherein the adjusting step (a1) and the mixing step (a2) are performed in parallel.

5. The method according to any one of claims 1 to 4, wherein the mixing step (a2) is performed under shear conditions with a shear rate of 5/s or more and 100,000/s or less.

6. The method according to any one of claims 1 to 5, wherein the adjusting step (a1) is performed under shear conditions with a shear rate of 5/s or more and 100,000/s or less.

7. The method according to any one of claims 1 to 6, wherein stirring power per unit liquid amount (Pv) in a mixer in the mixing step (a2) is 0.1 $kW/m^3$ or more and 100 $kW/m^3$ or less.

8. The method according to any one of claims 1 to 7, wherein stirring power per unit liquid amount (Pv) in a mixer in the adjusting step (a1) is 0.1 $kW/m^3$ or more and 100 $kW/m^3$ or less.

9. The method according to any one of claims 1 to 8, wherein

   the mixing step (a2) is performed using a reaction tank having an agitating impeller, or an agitating impeller type mixer, and
   a ratio (d/D) of an impeller diameter d to a tank inner diameter D of the reaction tank is 0.01 or more and less than 1.

10. The method according to any one of claims 1 to 9, wherein

    the mixing step (a2) is performed using a reaction tank having an agitating impeller, or an agitating impeller type mixer, and
    a ratio (h/H) of an agitating impeller height h to a tank height H of the reaction tank is 0.01 or more and less than 1.

11. The method according to any one of claims 1 to 10, wherein

    the adjusting step (a1) is performed using a reaction tank having an agitating impeller, or an agitating impeller type mixer, and
    a ratio (d/D) of an impeller diameter d to a tank inner diameter D of the reaction tank is 0.01 or more and less than 1.

12. The method according to any one of claims 1 to 11, wherein

    the adjusting step (a1) is performed using a reaction tank having an agitating impeller, or an agitating impeller type mixer, and
    a ratio (h/H) of an agitating impeller height h to a tank height H of the reaction tank is 0.01 or more and less than 1.

13. The method according to any one of claims 1 to 12, wherein a pressure in the adjusting step (a1) is 0 MPaG or more and 10 MPaG or less.

14. The method according to any one of claims 1 to 13, wherein a pressure in the mixing step (a2) is 0 MPaG or more and 10 MPaG or less.

15. The method according to any one of claims 1 to 14, wherein

    the mixing step (a2) is performed using a reaction tank having an agitating impeller, or an agitating impeller type mixer, and
    a peripheral speed of the agitating impeller is 0.01 m/s or more and 50 m/s or less.

16. The method according to any one of claims 1 to 15, wherein

    the adjusting step (a1) is performed using a reaction tank having an agitating impeller, or an agitating impeller type mixer, and
    a peripheral speed of the agitating impeller is 0.01 m/s or more and 50 m/s or less.

17. The method according to any one of claims 1 to 16, wherein the mixing step (a2) is performed at a temperature of 40°C or higher and 100°C or lower.

18. The method according to any one of claims 1 to 17, wherein a mixing amount of the decolorizing agent is 1 mg or more and 300 mg or less with respect to 1 g of the internal olefin sulfonate in the internal olefin sulfonate composition.

19. The method according to any one of claims 1 to 18, wherein the decolorizing agent is an oxidizing agent.

20. The method according to any one of claims 1 to 19, wherein the decolorizing agent is hydrogen peroxide.

21. The method according to any one of claims 1 to 20, wherein an aging step (b) is performed after the mixing step (a2).

22. The method according to claim 21, wherein a temperature in the aging step (b) is 40°C or higher and 120°C or lower.

23. The method according to claim 21 or 22, wherein a residence time in the aging step (b) is 10 minutes or more and 500 minutes or less.

24. The method according to any one of claims 21 to 23, wherein a pressure in the aging step (b) is 0 MPaG or more and 10 MPaG or less.

25. The method according to any one of claims 21 to 24, wherein a ratio ($H_1/D_1$) of a height $H_1$ of a device for performing the aging step (b) to a device diameter $D_1$ is 0.1 or more and 300 or less.

26. The method according to any one of claims 21 to 25, wherein a linear velocity of the internal olefin sulfonate composition in the aging step (b) is 0.001 mm/s or more and 1,000 mm/s or less.

27. The method according to any one of claims 1 to 26, wherein the alkali compound contains at least one alkali metal hydroxide selected from the group consisting of sodium hydroxide and potassium hydroxide.

28. The method according to any one of claims 1 to 27, wherein the internal olefin sulfonate contains at least one selected from the group consisting of an internal olefin sulfonate having 16 carbon atoms and an internal olefin sulfonate having 18 carbon atoms.

29. The method according to any one of claims 1 to 28, wherein an average double bond position of a raw material olefin of the internal olefin sulfonate is 3 or more and 5 or less.

30. The method according to any one of claims 1 to 29, wherein the concentration of the alkali compound in the internal olefin sulfonate composition before the decolorizing process (A) is performed is 0.010 mmol or more and 0.27 mmol or less with respect to 1 g of the internal olefin sulfonate in the internal olefin sulfonate composition.

31. The method according to claim 30, wherein

the internal olefin sulfonate composition is obtained by a method for producing an internal olefin sulfonate including a sulfonation process of sulfonating a raw material olefin, a neutralization process of neutralizing a sulfonated product obtained, and a hydrolysis process of hydrolyzing a neutralization product obtained, and the concentration of the alkali compound in the internal olefin sulfonate composition after the hydrolysis process is controlled by an adjusting method selected from a method for adjusting an amount of the alkali compound used in the neutralization process and a method for adjusting an amount of the alkali compound in the hydrolysis process.

32. The method according to any one of claims 21 to 26, wherein the aging step (b) is performed in a semi-batch method.

33. The method according to any one of claims 21 to 26, wherein the aging step (b) is performed in a continuous method.

34. The method according to any one of claims 1 to 33, wherein the adjusting step (a1) is performed in a continuous method with a circulation system, and a circulation ratio of the internal olefin sulfonate composition is greater than 0 times.

**EP 4 755 872 A1**

## INTERNATIONAL SEARCH REPORT

International application No.

**PCT/JP2024/027314**

| A. | CLASSIFICATION OF SUBJECT MATTER |
|---|---|

*C07C 303/44*(2006.01)i; *C07C 303/32*(2006.01)i; *C07C 309/20*(2006.01)i
FI:   C07C303/44; C07C309/20; C07C303/32

According to International Patent Classification (IPC) or to both national classification and IPC

| B. | FIELDS SEARCHED |
|---|---|

Minimum documentation searched (classification system followed by classification symbols)

C07C303/44; C07C303/32; C07C309/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2015-178467 A (L'OREAL) 08 October 2015 (2015-10-08)<br>example 1 | 1-34 |
| Y | JP 04-346970 A (KAO CORPORATION) 02 December 1992 (1992-12-02)<br>claim 1, paragraphs [0016]-[0022] | 1-34 |
| A | WO 2015/098415 A1 (KAO CORPORATION) 02 July 2015 (2015-07-02)<br>claims 1-8 | 1-34 |
| A | WO 2023/008464 A1 (KAO CORPORATION) 02 February 2023 (2023-02-02)<br>claims 1-7 | 1-34 |

☐ Further documents are listed in the continuation of Box C.      ☑ See patent family annex.

| | |
|---|---|
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **16 October 2024** | **29 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

73

## INTERNATIONAL SEARCH REPORT
### Information on patent family members

| | International application No. |
|---|---|
| | **PCT/JP2024/027314** |

| Patent document cited in search report | | | Publication date (day/month/year) | Patent family member(s) | | | Publication date (day/month/year) |
|---|---|---|---|---|---|---|---|
| JP | 2015-178467 | A | 08 October 2015 | US example 1 | 2017/0095410 | A1 | |
| | | | | WO | 2015/141699 | A1 | |
| | | | | EP | 3145474 | A1 | |
| | | | | KR | 10-2016-0133461 | A | |
| | | | | CN | 106102704 | A | |
| JP | 04-346970 | A | 02 December 1992 | (Family: none) | | | |
| WO | 2015/098415 | A1 | 02 July 2015 | US claims 1-14 | 2016/0332961 | A1 | |
| | | | | EP | 3088386 | A1 | |
| | | | | CN | 105849085 | A | |
| WO | 2023/008464 | A1 | 02 February 2023 | EP claims 1-7 | 4378918 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015178467 A
- WO 2011052732 A
- JP 11315061 A
- JP 49048409 B
- IT 1248079 A
- US 3257175 A